# EUROPEAN PATENT APPLICATION

(11) **EP 4 309 677 A1**
(43) Date of publication of application: **24.01.2024**
(21) Application number: 22770506.8
(22) Date of filing: 15.03.2022
(51) Int. Cl.: A61K 47/68, A61K 39/395, A61K 31/52, A61K 45/06, A61P 35/00, C07K 16/28, C12N 5/0783, C12N 5/0784

(54) **PD-L1 AND TLR7 DOUBLE-TARGETING NANOBODY COUPLING DRUG AND USE THEREOF IN ANTI-TUMOR**

(30) Priority: 16.03.2021 CN 202110282985
(71) Applicant: Shanghai Institute of Materia Medica, Chinese Academy of Sciences, Pudong, Shanghai 201203 (CN)
(72) Inventor: GONG, Likun, Shanghai 201203 (CN); YU, Xiaolu, Shanghai 201203 (CN); LONG, Yiru, Shanghai 201203 (CN); SUN, Jianhua, Shanghai 201203 (CN); TONG, Yongliang, Shanghai 201203 (CN); LIU, Tingting, Shanghai 201203 (CN)
(74) Representative: karo IP
(86) International application number: PCT/CN2022/080953
(87) International publication number: WO 2022/194153

(57) **Abstract**

Disclosed in the present invention are a use of a combination of an anti-PD-L1 nanobody and a TLR7 small molecule agonist in anti-tumor treatment, and a PD-L1 and TLR7 double-targeting nanobody coupling drug, a preparation method therefor, and a use thereof. Specifically, disclosed in the present invention are a use and solution of a combination of an anti-PD-L1 nanobody and a derived protein thereof as well as a TLR7 small molecule agonist and a derived compound thereof in anti-tumor treatment. Meanwhile, disclosed in the present invention are design, preparation, and identification solutions for a novel PD-L1 and TLR7 double-targeting-nanobody drug conjugate and a derived molecule thereof, and an effect of the novel PD-Ll and TLR7 double-targeting nanobody drug conjugate in anti-tumor treatment. The PD-L1 and TLR7 double-targeting nanobody drug conjugate of the present invention can yield a significant antineoplastic efficacy in various transplantation tumor models.

## Description

### Technical field

The present invention relates to the field of biomedicine, in particular to a PD-L1 and TLR7 double-targeting nanobody coupling drug and its use in anti-tumor therapy

### Background

Tumors achieve immune escape by upregulating the expression of immune checkpoint molecules such as PD-L1. A variety of antibody drugs targeting molecular targets of immune checkpoint such as PD-L1 have been developed for the treatment of tumors, but PD-L1 antibody and other immune checkpoint blocking therapies still face the problem of low response rate of patients. Combined treatment is an effective way to improve the anti-tumor efficacy and patient response rate of immune checkpoint blocking therapy. However, scientific, rational, safe and effective combined treatment still needs further research.

Toll-like receptor (TLR) is an important pattern recognition receptor in innate immunity. TLR receptor agonists can activate the innate immune response to assist the activation of adaptive immunity, and TLR7 agonists have been widely studied and used in anti-tumor therapy. TLR agonists are one of the potential combination targets for immune checkpoint blocking therapy.

Monoclonal antibody has made a breakthrough in tumor immunotherapy. However, traditional monoclonal antibodies have the disadvantages of high molecular weight, poor tissue penetration and high immunogenicity. Nanobody is the smallest antibody molecule at present, in addition to the specificity of monoclonal antibody, it has the advantages of strong tissue penetration, low immunogenicity, good stability, simple humanization and easy preparation. Nanobody coupling drugs developed on the basis of nanobody are a new form of drug molecules and have wide application prospects in drug delivery, *in vivo* imaging and anti-tumor therapy.

To sum up, there is an urgent need to develop a new and effective PD-L1 nanobody coupling drug in this field.

### Summary of the Invention

The object of the present invention is to provide a new and effective PD-L1 nanobody coupling drug.

The object of the present invention is to provide a combination of PD-L1 nanobody and TLR7 agonist for anti-tumor therapy and a double-targeted nanobody coupling drug of PD-L1 and TLR7.

Another object of the invention is to provide the application of PD-L1 and TLR7 double-targeted nanobody coupling drugs in tumor prevention and treatment, especially in tumors with low response rate of PD-L1 antibodies.

In a first aspect of the present invention, an antibody-drug conjugate or a pharmaceutically acceptable salt thereof is provided, and the structure of the antibody-drug conjugate is as shown in formula I:

Ab-(J-U)n ( I )

wherein,
Ab is a PD-L1 antibody;
U is each independently a TLR agonist;
J is a chemical bond or linker;
n is 0 or a positive integer;
"-"is a chemical bond or linker or connector.

In another preferred embodiment, the PD-L1 antibody comprises a monospecific antibody, a bispecific antibody, a multispecific antibody (such as a trispecific antibody).

In another preferred embodiment, the antibody includes: monoclonal antibody, single-chain antibody (scFv), nanobody.

In another preferred embodiment, the PD-L1 antibody comprises a monovalent, divalent or multivalent antibody.

In another preferred embodiment, the PD-L1 antibody comprises an antibody in the form of a polymer.

In another preferred embodiment, the PD-L1 antibody specifically binds to PD-L1.

In another preferred embodiment, the PD-L1 antibody comprises PD-L1 monovalent nanobodies, bivalent nanobodies and/or multivalent nanobodies.

In another preferred embodiment, the PD-L1 antibody includes a blocking type (which blocks the binding of PD-L1 and PD-1), a non-blocking type (which does not block the binding of PD-L1 and PD-1), or a combination thereof.

In another preferred embodiment, the PD-L1 antibody is a blocking antibody.

In another preferred embodiment, the PD-L1 antibody blocks the binding of PD-1 to PD-L1.

In another preferred embodiment, the PD-L1 is human PD-L1 or PD-L1 of a non-human mammal (such as mouse PD-L1).

In another preferred embodiment, the PD-L1 antibody is a human or non-human mammalian antibody.

In another preferred embodiment, the non-human mammals are selected from the following group: camels, alpacas, mice, crab-eating monkeys.

In another preferred example, the PD-L1 antibody is a PD-L1 nanobody or its derivative antibody.

In another preferred embodiment, the derived antibody is a modification of PD-L1 nanobodies, including but not limited to linking PD-L1 nanobodies to Fc fragments, human serum albumin, and polyethylene glycol PEG to form bivalent and/or multivalent antibodies.

In another preferred embodiment, the nanobody includes a humanized antibody, a camel antibody, a chimeric antibody.

In another preferred embodiment, the PD-L1 nanobody specifically binds to PD-L1, and the complementary determining region CDR of the VHH chain in the nanobody is selected from one or more of the following groups:
(1) CDR1 shown in SEQ ID NO: 2, CDR2 shown in SEQ ID NO: 3, CDR3 shown in SEQ ID NO: 4;
(2) CDR1 shown in SEQ ID NO:6, CDR2 shown in SEQ ID NO:7, CDR3 shown in SEQ ID NO:8;
(3) CDR1 shown in SEQ ID NO:10, CDR2 shown in SEQ ID NO:11, CDR3 shown in SEQ ID NO:12;
(4) CDR1 shown in SEQ ID NO:14, CDR2 shown in SEQ ID NO:15, CDR3 shown in SEQ ID NO:16; and
(5) CDR1 shown in SEQ ID NO:18, CDR2 shown in SEQ ID NO:19, CDR3 shown in SEQ ID NO:20.

In another preferred embodiment, the PD-L1 nanobody specifically binds to human PD-L1, and the complementary determining region CDR of the VHH chain in the nanobody is selected from one or more of the following groups:
(6) CDR1 shown in SEQ ID NO:22, CDR2 shown in SEQ ID NO:23, CDR3 shown in SEQ ID NO:24;
(7) CDR1 shown in SEQ ID NO:26, CDR2 shown in SEQ ID NO:27, CDR3 shown in SEQ ID NO:28;
(8) CDR1 shown in SEQ ID NO:30, CDR2 shown in SEQ ID NO:31, CDR3 shown in SEQ ID NO:32;
(9) CDR1 shown in SEQ ID NO:34, CDR2 shown in SEQ ID NO:35, CDR3 shown in SEQ ID NO:36;
(10) CDR1 shown in SEQ ID NO:22, CDR2 shown in SEQ ID NO:38, CDR3 shown in SEQ ID NO:39;
(11) CDR1 shown in SEQ ID NO:41, CDR2 shown in SEQ ID NO:42, CDR3 shown in SEQ ID NO:43;
(12) CDR1 shown in SEQ ID NO:45, CDR2 shown in SEQ ID NO:46, CDR3 shown in SEQ ID NO:47;
(13) CDR1 shown in SEQ ID NO:49, CDR2 shown in SEQ ID NO:50, CDR3 shown in SEQ ID NO:51;
(14) CDR1 shown in SEQ ID NO:53, CDR2 shown in SEQ ID NO:54, CDR3 shown in SEQ ID NO:55;
(15) CDR1 shown in SEQ ID NO:57, CDR2 shown in SEQ ID NO:50, CDR3 shown in SEQ ID NO:58; and
(16) CDR1 shown in SEQ ID NO:60, CDR2 shown in SEQ ID NO:61, CDR3 shown in SEQ ID NO:62.

In another preferred embodiment, any one of the above amino acid sequences further includes a derivative sequence that is optionally added, deleted, modified and/or substituted with at least one (such as 1-3, preferably 1-2, more preferably 1) amino acid and can retain the ability to bind to PD-L1.

In another preferred embodiment, the VHH chain of the anti-PD-L1 nanobodyis selected from the following group:
(a) Having an amino acid sequence as shown in SEQ ID NO:1, SEQ ID NO:5, SEQ ID NO:9, SEQ ID NO:13, or SEQ ID NO:17;
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1

In another preferred embodiment, the nanobody sequence comprises an amino acid sequence having a sequence similarity of at least 80%, preferably at least 90%, more preferably at least 95%, even more preferably at least 99%, with SEQ ID NO:1, SEQ ID NO:5, SEQ ID NO:9, SEQ ID NO:13, or SEQ ID NO:17.

In another preferred embodiment, the anti-PD-L1 nanobody also comprises specifically binding human PD-L1 nanobody.

In another preferred embodiment, the amino acid sequence of the VHH chain specifically binding to human PD-L1 nanobody is selected from the following group:
(a) Having the amino acid sequence shown in SEQ ID NO: 21, 25, 29, 33, 37, 40, 44, 48, 52, 56, 59;
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1

In another preferred embodiment, the nanobody sequence comprises an amino acid sequence having a sequence similarity of at least 80%, preferably at least 90%, more preferably at least 95%, even more preferably at least 99%, with SEQ ID NO: 21, 25, 29, 33, 37, 40, 44, 48, 52, 56 or 59.

In another preferred embodiment, the anti-PD-L1 nanobody also comprises humanized specifically binding human PD-L1 nanobody.

In another preferred embodiment, the amino acid sequence of the VHH chain of the humanized specifically binding to human PD-L1 nanobody is selected from the following group:
(a) Having the amino acid sequence shown in 63, 64, 65, 66, 67, 68, 69, 70, 71, 72, 73, 74, 75, 76, 77, 78, 79, 80, 81, 82, 83, 84, 85, 86, 87, 88, 89, 90, 91.
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1

In another preferred embodiment, the nanobody sequence comprises an amino acid sequence having a sequence similarity of at least 80%, preferably at least 90%, more preferably at least 95%, even more preferably at least 99%, with SEQ ID NO:63, 64, 65, 66, 67, 68, 69, 70, 71, 72, 73, 74, 75, 76, 77, 78, 79, 80, 81, 82, 83, 84, 85, 86, 87, 88, 89, 90, or 91.

In another preferred embodiment, the anti-PD-L1 nanobody also comprises affinity-matured specifically binding human PD-L1 nanobody.

In another preferred embodiment, the amino acid sequence of the VHH chain of the affinity-matured specifically binding to human PD-L1 nanobody is selected from the following group:
(a) Having the amino acid sequence shown in SEQ ID NO. 92, 93, 94, 95, 96, 97, 98, 99, 100, 101, 102, 103, 104, 105, 106, 107, 108, 109, 110, 111, 112, 113, 114, 115, 116, or 117.
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1

In another preferred example, the "affinity-matured" refers to an increase of at least 1, at least 2, at least 3, at least 4, at least 5, at least 6, at least 7, at least 8, at least 9, at least 10, at least 11, at least 12, at least 20, or at least 25 times in the affinity of the modified anti-human PD-L1 nanobody to PD-L1 compared to the pre-modified anti-human PD-L1 nanobody.

In another preferred embodiment, the nanobody sequence comprises an amino acid sequence having a sequence similarity of at least 80%, preferably at least 90%, more preferably at least 95%, even more preferably at least 99%, with SEQ ID NO: 92, 93, 94, 95, 96, 97, 98, 99, 100, 101, 102, 103, 104, 105, 106, 107, 108, 109, 110, 111, 112, 113, 114, 115, 116, or 117.

In another preferred embodiment, the complementary determination region CDR of the VHH chain in the nanobody is consisted of CDR1 shown in SEQ ID NO:2, CDR2 shown in SEQ ID NO:3, CDR3 shown in SEQ ID NO:4.

In another preferred embodiment, the VHH chain sequence in the nanobody is shown in SEQ ID NO: 1.

In another preferred embodiment, the TLR agonist is either a large molecule (protein or nucleic acid) or a small molecule agonist.

In another preferred embodiment, the TLR agonist includes, but is not limited to, a TLR1 agonist, a TLR2 agonist, a TLR3 agonist, a TLR4 agonist, a TLR5 agonist, a TLR6 agonist, a TLR7 agonist, a TLR8 agonist and a TLR9 agonist.

In another preferred embodiment, n is the average drug coupling quantity in the antibody-drug conjugate, preferably 1-9, preferably 2.5-6.5, and more preferably 3.5 ~5.5.

In another preferred embodiment, the TLR agonist is TLR7 agonist.

In another preferred embodiment, the TLR agonist does not have TLR8 agonist activity.

In another preferred embodiment, the TLR7 agonist is a host endogenous agonist or an exogenous agonist.

In another preferred embodiment, the TLR7 agonist is a small molecule agonist.

In another preferred embodiment, the TLR7 agonist includes: SZU-101:

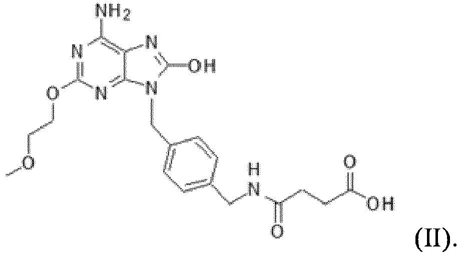

In another preferred embodiment, the TLR7 agonist is a derivative compound of SZU-101, including, but not limited to, replacement, modification, or deletion of one or more groups based on the SZU-101.

In another preferred embodiment, the TLR7 agonist is a multivalent compound of SZU-101.

In another preferred embodiment, the TLR7 agonist (eg, SZU-101) is attached to the terminal amino group or side chain amino group of the heavy chain constant region or the heavy chain variable domain (VHH) of the PD-L1 antibody.

In another preferred embodiment, the TLR7 agonist (eg, SZU-101) is attached to the mercapto group of the PD-L1 antibody.

In another preferred embodiment, the SZU-101 is attached to the amino group of the PD-L1 antibody and forms the structure shown in S1: 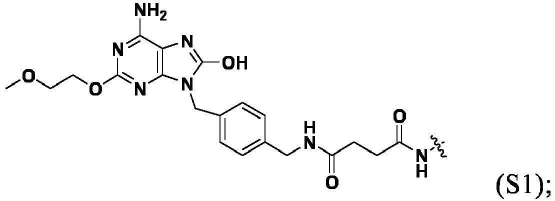 or
the SZU-101 is attached to the mercapto group of the PD-L1 antibody and forms the structure shown in S2:

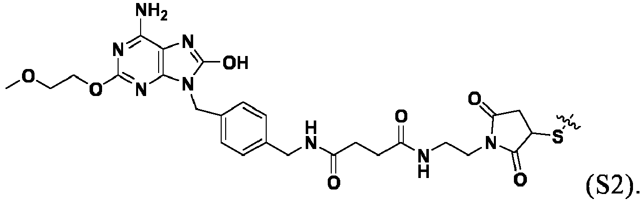

In another preferred embodiment, the TLR7 agonist is site-specific and/or randomly attached to the PD-L1 antibody (ie, in Formula I, the U is site-specific and/or randomly attached to Z).

In another preferred embodiment, the U is site-specific attached to Z.

In another preferred embodiment, the U is site-specific attached to amino acid sites of PD-L1 antibody Z selected from the group consisting of G, K, L, A, C or a combination thereof.

In another preferred embodiment, the chemical bond is polyethylene glycol PEG.

In another preferred embodiment, the chemical bond is a derivative compound of PEG, including, but not limited to, replacement, modification, or deletion of one or more groups performed on the basis of SZU-101.

In another preferred embodiment, the degree of polymerization of the PEG chemical bond is a positive integer greater than or equal to 1.

In another preferred embodiment, the antibody-drug conjugate improves the PD-L1 level of cells in tumor.

In another preferred embodiment, the antibody-drug conjugate actives immune cells.

In another preferred embodiment, the activation is *in vitro* activation.

In another preferred embodiment, the *in vitro* activation includes: culturing the immune cells for a period of time (such as 6-48 hours) in the presence of the antibody-drug conjugate to obtain the immuno-activiated immune cells.

In another preferred embodiment, the immune cells are selected from, but not limited to: CD8+T cells, natural killer cells NK, dendritic cells, lymphocytes, monocytes/macrophages, granulocytes, or a combination thereof.

In another preferred embodiment, the antibody-drug conjugate or a pharmaceutically acceptable salt thereof are used for preaparing a composition or formulation, which is used for:
(a) promoting maturation of dendritic cells;
(b) increasing the function of tumor infiltrating cytotoxic cells (CD8 + T cells and NK cells);
(c) promoting the expression of granzyme B and IFN-γ in tumor infiltrating cytotoxic cell;
(d) promoting re-polarization of tumor-associated macrophages;
(e) reducing the infiltration of TGF-β + macrophages;
(f) promoting the infiltration of IFN-γ + CD4 + T cells;
(g) promoting the expression of PD-L1 by macrophages in tumors;
(h) targeting and remodeling a tumor immune microenvironment;
(i) increasing the PD-L1 level of tumor cells; and/or
(j) treating the tumor with moderate-expression or low expression of PD-L1.

In another preferred embodiment, the remodeling a tumor immune microenvironment is an anti-tumor immune response coordinating innate and adaptive immune.

In another preferred embodiment, the remodeling a tumor immune microenvironment is to improve the infiltration of anti-tumor immune cells and reduce the proportion of immunosuppressive cells.

In another preferred embodiment, the anti-tumor immune cells include, but not limited to, CD8+T cells and NK cells secreting granzyme and IFN-γ, activated dendritic cells, CD4+T cells, M1 macrophages.

In another preferred embodiment, the immunosuppressive cells include, but not limited to, M2 macrophages, Treg cells, leucocytes secreting TGF-β.

In another preferred embodiment, the PD-L1 level comprises a cell surface PD-L1 level and an intracellular PD-L1 level.

In another preferred embodiment, the tumor with low expression of PD-L1 is a solid tumor or a hemangioma.

In the second aspect of the present invention, it provides a pharmaceutical composition, wherein the pharmaceutical composition comprises:
(a) a antibody-drug conjugate or a pharmaceutically acceptable salt thereof according to the first aspect of the present invention; and
(b) a pharmaceutically acceptable carrier.

In another preferred embodiment, the pharmaceutical composition also comprises:
(c) other biologically active drugs, such as those used to treat tumors.

In another preferred embodiment, the other biologically active drugs promote the anti-tumor function of CD8+T cells and NK cells.

In another preferred embodiment, the pharmaceutical composition includes single drugs, compound drugs, or synergistic drugs.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spray and atomization inhalation.

In another preferred embodiment, the administration method of the pharmaceutical composition comprises: after co-culturing the pharmaceutical composition and immune cells (such as dendritic cells, natural killer cells, lymphocytes, mononuclear/macrophages, granulocytes, etc.), separating immune cells for *in-vivo* backhaul.

In another preferred embodiment, the dosage form of the pharmaceutical composition is selected from the group consisting of liquid, solid, or gel.

In another preferred embodiment, the pharmaceutical composition is used to treating tumors.

In another preferred embodiment, the pharmaceutical composition is used for treating tumor with low expression of PD-L1.

In another preferred embodiment, "low expression of PD-L1" means that the amount E1 of PD-L1 expressed by the tumor is lower than the amount E0 of normal tumor expression PD-L1, preferably E1/E0 ≤ 1/2, more preferably ≤ 1/3, and more preferably ≤ 1/4.

In another preferred embodiment, the tumors include, but are not limited to, breast cancer, liver cancer, gastric cancer, colorectal cancer, leukemia, lung cancer, kidney tumor, intestinal cancer, prostate cancer, colorectal cancer, prostate cancer, cervical cancer, lymphoma, bone cancer, adrenal tumor, or bladder tumor.

In the third aspect of the present invention, it provides an immune conjugate, which comprises:
(a) an antibody-drug conjugate according to the first aspect of the present invention; and
(b) other coupling parts.

In another preferred embodiment, the other coupling parts are selected from the group consisting of a small molecule compound, PEG, fluorescein, a radioisotope, a contrast agent, a fatty acid chain, a protein fragment, or a combination thereof.

In another preferred embodiment, the components (a) and (b) are operatively connected.

In another preferred embodiment, the the coupling parts comprise a chemical marke and a biomarker.

In another preferred embodiment, the chemical marker is selected from an isotope, an immunotoxin, and/or a chemical drug.

In another preferred embodiment, the biomarker is selected from biotin, avidin or an enzyme tag.

In another preferred embodiment, the small molecule compound is selected from drugs or toxins for treating tumors or autoimmune diseases.

In another preferred embodiment, the radioisotope comprises:
(i) a diagnostic isotope, which is selected from the group consisting of Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope, which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the radioisotope includes, but is not limited to, iodine 131, indium 111, and Lutetium 177.

In another preferred embodiment, the contrast agent is used for MRI or CT.

In another preferred embodiment, the protein fragment includes, but is not limited to, an antibody Fc, biotin, avidin, HRP, an antibody, an enzyme, a cytokine and other bioactive proteins or polypeptides.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), or a nanoparticle in any form.

In the fourth aspect of the invention, a fusion protein is provided, which comprise:
(a) a PD-L1 nanobody according to the eighth aspect of the present invention; and
(b) an optional polypeptide molecules and protein fragments with therapeutic functions.

In another preferred embodiment, the polypeptide molecules or fragments with therapeutic functions include, but are not limited to: polypeptide molecules or fragments targeting PD-1, IL-4R, IL-4Rα, TNF-α, VEGF, 4-1BB, CD47, TIM3, CTLA4, IL-17A, CD19, CD22, CD28, CD38, CD40, CD47, B7-H3, TSLP, BCMA, GLP-1, Trop2, TIGIT, LAG-3, FGL1, HER2.

In another preferred embodiment, the polypeptide molecules or fragments with therapeutic functions include but are not limited to: insulin, IL-2, interferon, calcitonin, GHRH peptide, intestinal peptide analog, albumin, antibody fragments, cytokines.

In another preferred embodiment, the polypeptide molecules or fragments with therapeutic functions comprise single-chain antibody (scFV), double-stranded antibody, monoclonal antibody, or chimeric antibody.

In another preferred embodiment, the fusion protein also comprises a tag sequence for assisting expression and/or purification.

In another preferred embodiment, the tag sequence is selected from the following group: a 6Histag, a GGGS sequence, and an FLAG tag.

In another preferred embodiment, the fusion protein comprises a bispecific antibody and a chimeric antibody.

In the fifth aspect of the present invention, it provides a multispecific antibody, wherein the multispecific antibody comprises:
(a) a PD-L1 nanobody according to the eighth aspect of the present invention; and
(b) an optional antibody molecule targeting the second antigen.

In another preferred embodiment, the multispecific antibody comprises a second antigen binding region targeting a target selected from the following group: PD-1, IL-4 R, IL-4 R alpha, TNF-alpha, VEGF, 4 -1 BB, CD47, TIM 3, CTLA4, IL-17 A, CD19, CD22, CD28, CD38, CD40, CD47, B7 -H3, TSLP, BCMA, GLP-1, TROP 2, TIGIT, LAG-3, FGL1, HER2, or a combination thereof.

In another preferred embodiment, the second antigen-binding region is a nanobody.

In another preferred embodiment, the multispecific antibody comprises one or more second antigen-binding regions.

In another preferred embodiment, the multispecific antibody further comprises an Fc segment of the antibody.

In the sixth aspect of the present invention, it is provided a method for preparing antibody-drug conjugate according to the first aspect of the present invention, which comprises the steps:
configuring a reaction system, the reaction system comprising an antibody and a free drug molecule, and then performing a coupling reaction to obtain the antibody-drug conjugate, wherein the drug molecule comprises a TLR agonist and a linker.

In another preferred embodiment, the reaction time is 3h-10h.

In another preferred embodiment, the molar ratio of the antibody to the drug molecule is 1-2: 3-20; preferably 1: 6-10.

In another preferred embodiment, SZU-101, EDCI and NHS are dissolved in DMSO and stirred for three hours at room temperature to prepare SZU-101-NHS active ester, and the PD-L1 nanobody and the SZU-101-NHS active ester are stirred and reacted at 4°C at a molar ratio of 1: 10 for 4 hours to prepare a nanobody coupling drug.

In the seventh aspect of the present invention, it is provided a method for preventing or treating tumors, by administrating the nanobody conjugate drug according to the first aspect of the present invention to a subject in need thereof.

In another preferred embodiment, the tumor is a tumor expressing PD-L1.

In another preferred embodiment, the tumor is selected from the group consisting of high-expression PD-L1 tumors, moderate-expression PD-L1 tumors, and low-expression PD-L1 tumors.

In another preferred embodiment, the tumor is a moderate-expression PD-L1 tumors or a low-expression PD-L1 tumor.

In another preferred embodiment, the tumor is a low-expression PD-L1 tumor.

In another preferred embodiment, the "high-expression PD-L1" means that the ratio of the amount E1 of PD-L1 expressed by the tumor to the amount E0 expressed by the normal tumor (E1/E0) is > 1, more preferably ≥ 1.5, and more preferably ≥ 2.0.

In another preferred embodiment, the "moderate-expression PD-L1" means that the ratio of the amount E1 of PD-L1 expressed by the tumor to the amount E0 expressed by the normal tumor (E1/E0) is 0.5 -1.1, more preferably 0.7 -1.0, and more preferably 0.8 -0.9.

In another preferred embodiment, the "low-expression PD-L1" means that the ratio of the amount E1 of PD-L1 expressed by the tumor to the amount E0 expressed by the normal tumor (E1/E0) is ≤ 1/2, more preferably ≤ 1/3, and more preferably ≤ 1/4.

In another preferred embodiment, the tumors include, but are not limited to, breast cancer, liver cancer, gastric cancer, colorectal cancer, leukemia, lung cancer, kidney tumor, intestinal cancer, prostate cancer, colorectal cancer, prostate cancer, cervical cancer, lymphoma, bone cancer, adrenal tumor, or bladder tumor.

In the eighth aspect of the present invention, it is provided a PD-L1 nanoboy, which can specifically bind to PD-L1, and the complementary determination region CDR of the VHH chain in the nanobody is one or more selected from the group consisting of:
(1) CDR1 shown in SEQ ID NO: 2, CDR2 shown in SEQ ID NO: 3, CDR3 shown in SEQ ID NO: 4;
(2) CDR1 shown in SEQ ID NO:6, CDR2 shown in SEQ ID NO:7, CDR3 shown in SEQ ID NO:8;
(3) CDR1 shown in SEQ ID NO:10, CDR2 shown in SEQ ID NO:11, CDR3 shown in SEQ ID NO:12;
(4) CDR1 shown in SEQ ID NO:14, CDR2 shown in SEQ ID NO:15, CDR3 shown in SEQ ID NO:16; and
(5) CDR1 shown in SEQ ID NO:18, CDR2 shown in SEQ ID NO:19, CDR3 shown in SEQ ID NO:20.

In another preferred example, the PD-L1 nanobody specifically binds to PD-L1, and the complementary determining region CDR of the VHH chain in the nanobody is selected from one or more of the following groups:
(6) CDR1 shown in SEQ ID NO:22, CDR2 shown in SEQ ID NO:23, CDR3 shown in SEQ ID NO:24;
(7) CDR1 shown in SEQ ID NO:26, CDR2 shown in SEQ ID NO:27, CDR3 shown in SEQ ID NO:28;
(8) CDR1 shown in SEQ ID NO:30, CDR2 shown in SEQ ID NO:31, CDR3 shown in SEQ ID NO:32;
(9) CDR1 shown in SEQ ID NO:34, CDR2 shown in SEQ ID NO:35, CDR3 shown in SEQ ID NO:36;
(10) CDR1 shown in SEQ ID NO:22, CDR2 shown in SEQ ID NO:38, CDR3 shown in SEQ ID NO:39;
(11) CDR1 shown in SEQ ID NO:41, CDR2 shown in SEQ ID NO:42, CDR3 shown in SEQ ID NO:43;
(12) CDR1 shown in SEQ ID NO:45, CDR2 shown in SEQ ID NO:46, CDR3 shown in SEQ ID NO:47;
(13) CDR1 shown in SEQ ID NO:49, CDR2 shown in SEQ ID NO:50, CDR3 shown in SEQ ID NO:51;
(14) CDR1 shown in SEQ ID NO:53, CDR2 shown in SEQ ID NO:54, CDR3 shown in SEQ ID NO:55;
(15) CDR1 shown in SEQ ID NO:57, CDR2 shown in SEQ ID NO:50, CDR3 shown in SEQ ID NO:58; and
(16) CDR1 shown in SEQ ID NO:60, CDR2 shown in SEQ ID NO:61, CDR3 shown in SEQ ID NO:62.

In another preferred embodiment, any one of the above amino acid sequences further includes a derivative sequence that is optionally added, deleted, modified and/or substituted with at least one (such as 1-3, preferably 1-2, more preferably 1) amino acid and can retain the ability to bind to PD-L1.

In another preferred embodiment, the VHH chain of the anti-PD-L1 nanobody is selected from the following group:
(a) Having the amino acid sequence shown in SEQ ID NO:1, SEQ ID NO:5, SEQ ID NO:9, SEQ ID NO:13, or SEQ ID NO:17;
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1

In another preferred embodiment, the nanobody sequence comprises an amino acid sequence having a sequence similarity of at least 80%, preferably at least 90%, more preferably at least 95%, even more preferably at least 99%, with SEQ ID NO:1, SEQ ID NO:5, SEQ ID NO:9, SEQ ID NO:13, or SEQ ID NO:17.

In another preferred embodiment, the anti-PD-L1 nanobody also comprises specifically binding human PD-L1 nanobody.

In another preferred embodiment, the amino acid sequence of the VHH chain specifically binding to human PD-L1 nanobody is selected from the following group:
(a) having the amino acid sequence shown in SEQ ID NO: 21, 25, 29, 33, 37, 40, 44, 48, 52, 56, 59;
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1.

In another preferred embodiment, the nanobody sequence comprises an amino acid sequence having a sequence similarity of at least 80%, preferably at least 90%, more preferably at least 95%, even more preferably at least 99%, with SEQ ID NO: 21, 25, 29, 33, 37, 40, 44, 48, 52, 56, or 59.

In another preferred embodiment, the anti-PD-L1 nanobody also comprises humanized specifically binding human PD-L1 nanobody.

In another preferred embodiment, the amino acid sequence of the VHH chain of the humanized specifically binding to human PD-L1 nanobody is selected from the following group:
(a) having the amino acid sequence shown in 63, 64, 65, 66, 67, 68, 69, 70, 71, 72, 73, 74, 75, 76, 77, 78, 79, 80, 81, 82, 83, 84, 85, 86, 87, 88, 89, 90, 91.
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1.

In another preferred embodiment, the nanobody sequence comprises an amino acid sequence having a sequence similarity of at least 80%, preferably at least 90%, more preferably at least 95%, even more preferably at least 99%, with SEQ ID NO:63, 64, 65, 66, 67, 68, 69, 70, 71, 72, 73, 74, 75, 76, 77, 78, 79, 80, 81, 82, 83, 84, 85, 86, 87, 88, 89, 90, or 91.

In another preferred embodiment, the anti-PD-L1 nanobody also comprises affinity-matured specifically binding human PD-L1 nanobody.

In another preferred embodiment, the amino acid sequence of the VHH chain of the affinity-matured specifically binding to human PD-L1 nanobody is selected from the following group:
(a) having the amino acid sequence shown in 92, 93, 94, 95, 96, 97, 98, 99, 100, 101, 102, 103, 104, 105, 106, 107, 108, 109, 110, 111, 112, 113, 114, 115, 116, or 117.
(b) a derivative polypeptide or active fragment formed by one or more amino acids addition, one or more amino acids substitution, or 1-3 amino acids deletion to the amino acid sequence in (a), and the derivative polypeptide or active fragment retains a specific binding capability with PD-L1.

In another preferred embodiment, the nanobody sequence comprises an amino acid sequence having a sequence similarity of at least 80%, preferably at least 90%, more preferably at least 95%, even more preferably at least 99%, with SEQ ID NO: 92, 93, 94, 95, 96, 97, 98, 99, 100, 101, 102, 103, 104, 105, 106, 107, 108, 109, 110, 111, 112, 113, 114, 115, 116, or 117.

In another preferred example, the "affinity-matured" refers to an increase of at least 1, at least 2, at least 3, at least 4, at least 5, at least 6, at least 7, at least 8, at least 9, at least 10, at least 11, at least 12, at least 20, or at least 25 times in the affinity of the modified anti-human PD-L1 nanobody to PD-L1 compared to the pre-modified anti-human PD-L1 nanobody.

In the ninth aspect of the present invention, it is provided a medicine box, wherein the medicine box comprises:
(1) a first container, and a PD-L1 nanobody according to the eighth aspect of the present invention located in the first container, and a pharmaceutically acceptable carrier;
(2) a second container, and a TLR7 agonist located in the second container, and a pharmaceutically acceptable carrier;
and (3) an optional use of the description.

It is to be understood that the various technical features of the present invention mentioned above and the various technical features specifically described hereinafter (as in the Examples) may be combined with each other within the scope of the present invention to constitute a new or preferred technical solution, which will not be repeated one by one herein, due to space limitations.

### Description of Drawings

FIG. 1 shows an EC50 curve of ELISA assay nanobody Nb16 and PD-L1 binding.
FIG. 2 shows an IC50 curve for ELISA assay nanobody Nb16 blocking PD-1 and PD-L 1 binding.
FIG. 3 shows an *in vivo* anti-tumor effect of a PD-L1 nanobody and a TLR7 agonist in combined treatment, where 3A is a schematic diagram of experimental tumor and the number of administration days; 3B is a mouse tumor growth curve; 3C is an end tumor weight; and 3D is an end tumor anatomical photograph.
FIG. 4 shows the structural schematic (4A) of PD-L1 and TLR7 dual targeting nanobody coupling drug and mass spectrometry identification (4B and 4C) before and after coupling.
FIG. 5 shows an EC50 curve of the combination of PD-L1 and TLR7 double-targeting nanobody conjugate drugs Nb16 - SZU-101 and PD-L1 in a flow cytometry, wherein the nanobody NB 16 is a control.
FIG. 6 shows the IC50 curve of PD-L1 and TLR7 double-targeting nanobody coupling drug Nb16-SZU-101 blocking PD-1 and PD-L1 binding in flow cytometry, where nanobody NB 16 is a control.
FIG. 7 shows the anti-tumor effect of PD-L1 and TLR7 double--targeting nanobody coupling drug Nb 16-SZU-101 in an induced CT26 tumor model, wherein 7A is a mouse tumor growth curve; 7B is an end tumor weight; and 7C is an end tumor anatomical photograph.
FIG. 8 shows an anti-tumor effect of PD-L1 and TLR7 double-1 targeting nanobody coopling drugs Nb16-SZU-101 in an uninduced and induced CT26 tumor model, wherein 8A is an IFN-γ-induced (PD-L1 high expression) and a comparison diagram of the expression quantity of PD-L1 on the CT 26 cell surface which is not induced by IFN-γ (PD-L1 low expression); 8B is a mouse tumor growth curve; 8C is an end tumor weight; and 8D is an end tumor anatomical photograph.
FIG. 9 shows the anti-tumor effect of PD-L1 and TLR7 double-targeting nanobody conjugate drugs Nb16 - SZU-101 in a B16 tumor model, wherein 9A is a schematic diagram of PD-L1 expression of B16-F10 cells that do not induce low-expression PD-L1, BLANK is a blank control group, and Isotype Control is the same type control group; 9B is a mouse tumor growth curve; 9C is an end tumor weight; and 9D is an end tumor anatomical photograph.
FIG. 10 shows a tumor inhibition effect of PD-L1 and TLR7 doubing-targeting nanobody conpling drugs Nb16-SZU-101 in a CT26 early model (tumor volume < 50 mm³) and a late model (tumor volume > 200 mm³), wherein 10A is a mouse tumor growth curve in an early model, and 10B is a mouse tumor survival curve in an early model; 10C is a mouse tumor growth curve in the late model, and 10D is a mouse tumor survival curve in the late model; and 10E is a tumor growth curve of mice in the re-tumor model.
FIG. 11 shows the PD-L1 and TLR7 double-targeting nanobody coupling drug Nb 16-SZU-101 to reshape tumor immune microenvironment, wherein total is the total amount of cells, mFc is the same type control group (mFc), and Nb 16-SZU -101 is a nanobody coupling drug group.
FIG. 12 shows that the PD-L1 and TLR7 double-targeted nanobody conupling drug Nb16-SZU-101 exhibits anti-tumor effects via CD8+T cells and NK cells when administered; where 12A is a tumor growth curve; 12B is an end tumor weight; and 12C is an end tumor photograph.
FIG. 13 shows the human PD-L1 binding activity of the nanobody of the candidate anti-human PD-L1, wherein blank is a negative control.
FIG. 14 shows the blocking activity determination of the nanobody of candidate anti-human PD-L1 on human PD-1/PD-L1 binding, wherein blank and blank + ligand are control groups.

### Detailed description

Through extensive and deep research, a PD-L1 nanobody is screened and identified for the first time, and a PD-L1 and TLR7 doubling-targeting nanobody coupling drug is developed. Specifically, it is found in various mouse transplantation tumor models that the doubling-targeting nanobody coupling drug of the present invention has excellent anti-tumor activity. In addition, the present invention also unexpectedly finds that the dual-targeting nanobody coupling drug of the present invention promotes the expression of PD-L1 in tumor macrophages, and mainly exerts in vivo anti-tumor activity through CD8 + T cells and NK cells, which is beneficial to the treatment of " cold " tumors with low expression of PD-L1 molecules. The PD-L1 and TLR7 dual-targeting nanobody coupling drug developed by the present invention shows a prominent anti-tumor effect and a novel action mechanism, and has clinical development and application values. The present invention has been completed on the basis of this.

### TLR receptor and TLR receptor agonist

As used herein, the term " TLR receptor " refers to a Toll-like receptor, is an important inherent immune mode recognition receptor in an organism immune system, and can specifically recognize relatively conservative antigen molecules (or referred to as pathogen-related molecular patterns) in a pathogenic microorganism evolution process, so as to realize effective detection and inherent immune response induction of pathogenic microorganism invasion. Ten TLR receptors have been found in the human body, ie, TLR1-TLR10 , wherein TLR3, TLR7, TLR8, TLR9 are located on the endosome of the cell, on the lysosomal membrane, and the rest are located on the cytoplasmic membrane. In this embodiment of the present invention, TLR7 is preferred as one of the drug molecule targets. The natural ligand of the TLR7 molecule is a single-stranded linear RNA.

As used herein, the term "TLR receptor agonist" refers to a macromolecule (protein or nucleic acid) or a small molecule agonist that can specifically bind and activate a TLR receptor, promote transduction of downstream signals of a TLR receptor, and achieve activation of intrinsic immune cells. In an embodiment of the present invention, the TLR7 agonist is preferred to construct the nanobody coupling drug. In addition to the SZU-101 applied in the embodiments of the present invention, the available TLR7 agonist also includes imiquimod, R848, and the like.

As used herein, the terms "nanobody of the present invention", "the nanobody targeting PD-L1 of the present invention", "anti-PD-L1 nanobody of the present invention" can be used interchangeably to refer to a nanobody that specifically recognize and bind to PD-L1( including human or mouse PD-L1). The particularly preferred one is the nanoantibody (Nb16) with the amino acid sequence of the VHH chain shown in SEQ ID NO: 1.

As used herein, the terms " nanobody coupling drug of the present invention ", "double-targeting nanobody coupling drug of the present invention ", " PD-L1 and TLR7 double-targeting nanobody coupling drugs of the present invention ", can be used interchangeably, both referring to novel drug molecules that specifically recognize and bind to PD-L1 (including human or mouse PD-L1) and derived protein coupled TLR7 agonists. The nanobody in the nanobody coupling drug is particularly preferred that the nanobody with the amino acid sequence of the VHH chain as shown in SEQ ID NO: 1.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of a light chain and a heavy chain.

As used herein, the terms "single domain antibody (sdAb or VHH)", "nanobody" have the same meaning and refer to cloning the variable region of the heavy chain of the antibody, constructing a nanobody composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

Nanobody/single-domain antibody (Nanobody) is used as a novel small molecule antibody fragment, and is obtained by cloning a heavy chain variable region (VHH) of a camel natural heavy chain antibody. Nanobody (Nb) has excellent biological characteristics, has a molecular weight of 12 -15 kDa, is one tenth of a complete antibody, and has good tissue penetrability, high specificity and good water solubility. Due to the special structural properties of the antibody, the advantages of a traditional antibody and a small molecule drug are both achieved, the defects of long development cycle, low stability, harsh preservation conditions and the like of a traditional antibody are almost perfectly overcome, the traditional antibody gradually becomes an emerging force in new generation of antibody treatment, and a wide application prospect is displayed in immunodiagnosis and treatment.

As used herein, the term "variable" means that certain portion of the variable region in an antibody differ in sequence, which is responsible for the binding and specificity of various specific antibodies to their specific antigen. However, the variability is not distributed evenly throughout the variable regions of an antibody. It is concentrated in three fragments called complementarity determination regions (CDR) or hypervariable regions in light chain and heavy chain variable regions. The more conserved part of variable region is called the framework regions (FR). The variable regions of the natural heavy and light chains each contain four FR regions, which are roughly in the β-folded configuration, connected by the three CDRs that form the connecting loop, and in some cases may form a partly folded structure. The CDRs in each chain get close through the FR regions and together with the CDRs of the other chain form the antigen-binding site of the antibody (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

As known to those skilled in the art, an immunoconjugates and the fusion expression product includes: a drug, a toxin, a cytokine, a radionuclide, an enzyme and other diagnostic or therapeutic molecules that bind to the antibody or fragment thereof of the present invention to form a conjugate.

As used herein, the terms " nanobody coupling drug "and"nanobody drug conjugate" may be used interchangeably. As is known to those skilled in the art, the nanobody conjugate is in the form of a special antibody drug conjugate drug, which is in the form of a drug molecule formed by coupling a nanobody or a derivative protein, a toxin, a cell factor, a radionuclide, an enzyme and other diagnostic or therapeutic molecules, and can be used for tumor treatment, drug delivery, *in-vivo* imaging, etc., and has a broad clinical application value.

As used herein, the terms "heavy chain variable region" and "V_{H}"can be used interchangeably.

As used herein, the terms "variable region" and "complementarity determine region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and the heavy chain constant region.

In the present invention, the terms "antibody of the present invention", "protein of the present invention", or "polypeptide of the present invention" may be used interchangeably and refer to a polypeptide that specifically binds to PD-L1, such as a protein or polypeptide having a heavy chain variable region. They can contain or do not contain starting methionine.

The invention also provides other proteins or fusion expression products having the antibody of the present invention. Specifically, the present invention includes any protein or protein conjugate and fusion expression product (i.e., immunoconjugate and fusion expression product) having a heavy chain containing variable regions, as long as the variable region is the same as or has at least 90% homology with the variable regions of the heavy chain of the antibody of the present invention, preferably at least 95% homology.

In general, the antigen-binding properties of an antibody can be described by the three specific regions located in the variable regions of the heavy chains, called complementary determining regions (CDR), which divide this segment into 4 framework regions (FR), and the amino acid sequences of the four FRs are relatively conservative and do not directly participate in the binding reaction. These CDRs form a loop structure, and the β-sheets formed by the FRs in between are spatially close to each other, and the CDRs on the heavy chain and the CDRs on the corresponding light chain constitute the antigen-binding site of the antibody. The amino acid sequences of antibodies of the same type can be compared to determine which amino acids constitute the FR or CDR regions.

The variable regions of the heavy chains of the antibody of the present invention are of particular interest because at least part of them involve binding antigens. Therefore, the present invention includes those molecules with a CDR-bearing antibody heavy chain variable region, as long as their CDR has more than 90% (preferably more than 95%, most preferably more than 98%) homology with the CDR identified here.

The present invention includes not only intact antibodies, but also fragments of immunologically active antibodies or fusion proteins formed by antibodies with other sequences. Thus, the present invention also includes fragments, derivatives and analogs of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having PD-L1 binding activity and comprising the above-mentioned CDR regions. The term also includes variant forms of polypeptides comprising the CDR regions described above that have the same function as the antibody of the present invention. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C- terminal and/or N-terminal. For example, in the art, substitutions with amino acids of similar properties generally do not alter the function of the protein. For another example, addition of one or more amino acids to the C-terminal and/or N-terminal usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as fusion proteins containing nanobodies or fragments thereof. In addition to the almost full-length polypeptide, the present invention also includes fragments of the nanobody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by replacing at most 10, preferably at most 8, more preferably at most 5, and most preferably at most 3 amino acids with amino acids of similar properties as compared with the amino acid sequence of the antibody of the present invention. These conservative variant polypeptides are best produced by amino acid substitution according to Table A.

**Table A**

| Initial residue | | Representative substitution | Preferred substitution | |
|---|---|---|---|---|
| | Ala (A) | Val; Leu; Ile | | Val |
| | Arg (R) | Lys; Gln; Asn | | Lys |
| | Asn (N) | Gln; His; Lys; Arg | | Gln |
| | Asp (D) | Glu | | Glu |
| | Cys (C) | Ser | | Ser |
| | Gln (Q) | Asn | | Asn |
| | Glu (E) | Asp | | Asp |
| | Gly (G) | Pro; Ala | | Ala |
| | His (H) | Asn; Gln; Lys; Arg | | Arg |
| | Ile (I) | Leu; Val; Met; Ala; Phe | | Leu |
| | Leu (L) | Ile; Val; Met; Ala; Phe | | Ile |
| | Lys (K) | Arg; Gln; Asn | | Arg |
| | Met (M) | Leu; Phe; Ile | | Leu |
| | Phe (F) | Leu; Val; Ile; Ala; Tyr | | Leu |
| | Pro (P) | Ala | | Ala |
| | Ser (S) | Thr | | Thr |
| | Thr (T) | Ser | | Ser |
| | Trp (W) | Tyr; Phe | | Tyr |
| | Tyr (Y) | Trp; Phe; Thr; Ser | | Phe |
| | Val (V) | Ile; Leu; Met; Phe; Ala | | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA can be single stranded or double stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridize to the above-mentioned sequence and have at least 50%, preferably at least 70%, and more preferably at least 80% identity between the two sequences. In particular, the present invention relates to a polynucleotide that is hybridizable to the polynucleotide of the present invention under strict conditions. In the present invention, "strict conditions" refers: (1) hybridization and elution at lower ionic strength and higher temperature, such as 0.2 × SSC,0.1% SDS, 60 °C; or (2) hybridiztion with denaturing agent, such as 50%(v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments followed by ligation. In addition, the coding sequence of the heavy chain and the expression tag (such as 6His) can be fused together to form a fusion protein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to enable them to express proteins.

Host cells may be prokaryotic cells, such as bacterial cells; or lower eukaryotic cells, such as yeast cells; or higher eukaryotic cells, such as mammalian cells. Representative examples include: Escherichia coli, Streptomyces; bacterial cells of Salmonella typhimurium; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as Escherichia coli, the competent cells capable of absorbing DNA can be harvested after the exponential growth period and treated with CaCl₂, the steps used are well known in the art. Another method is to use MgCl₂. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for a period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. and include, but are not limited to, conventional renaturation treatment, treatment by protein precipitant (such as salt precipitation), centrifugation, cell lysis by osmosis, sonication, supercentrifugation, molecular sieve chromatography (gel chromatography), adsorption chromatography, ion exchange chromatography, high performance liquid chromatography (HPLC), and any other liquid chromatography, and the combination thereof.

The antibody of the present invention can be used alone, or can be combined or coupled with a detectable label (for diagnostic purposes), a therapeutic agent, a PK (protein kinase) modifying moietiy, or any combination of these substances.

A detectable marker for diagnostic purposes includes, but is not limited to, a fluorescent or luminescent label, a radioactive label, a MRI (magnetic resonance imaging) or CT (electronic computer tomography) contrast agent, or an enzyme capable of producing a detectable product.

Therapeutic agents that may be bound or coupled with the antibodies of the present invention include, but are not limited to:1. a radionuclide; 2. a biological toxin; 3. a cytokine such as IL-2, etc; 4. a gold nanoparticle/nanorod; 5. a viral particle; 6. a liposome; 7. a nanomagnetic particle; 8. a drug-activating enzyme (e. g., DT-myoflavase (DTD) or biphenyl hydrolase-like protein (BPHL)); 9. a chemotherapeutic agent (e.g, cisplatin) or a nanoparticle in any form, etc.

### Antibody-Drug Conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug or drug with immune-promoting properties.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is acetate, nitrogen ions or nitrate; and polymethylene dimethyl sulfide thiosulfonate. The linker may include, for example, a maleimide linked to an antibody via thiosuccimide.

It should be understood that a drug usually may be any cytotoxic, cytostatic or immunosuppressive drug. In the present invention, the drug is a drug that can activate or promote an immune response, such as activating an innate immune response to assist the activation of adaptive immunity. In a specific embodiment, the drug is TLR receptor agonist.

In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, TLR1 agonist, TLR2 agonist, TLR3 agonist, TLR4 agonist, TLR5 agonist, TLR6agonist, TLR7 agonist, TLR8 agonist and TLR9 agonist, for example, SZU-101, Miquimod, R848, CpG et al. In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups is selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows:

Ab-(J-U)n ( I )

wherein,
Ab is a PD-L1 antibody;
U is each independently a TLR agonist;
J is a chemical bond or linker;
n is 0 or a positive integer;
"-"is a chemical bond or linker or connector.

### Use

The present invention provides use of the antibody according to the present invention, for example, for preparation of a diagnostic agent, or for preparation of a medicament for preventing and/or treating a PD-L1 associated disease. The PD-L1 associated disease includes inflammatory diseases, autoimmune diseases, etc., including but not limited to breast cancer, liver cancer, gastric cancer, colorectal cancer, leukemia, lung cancer, kidney cancer, intestinal cancer, prostate cancer, colorectal cancer, prostate cancer, cervical cancer, lymph cancer, bone cancer, adrenal tumor, or bladder tumor.

It should be understood that cancers that do not respond to treatment with one or more checkpoint inhibitors (e.g., antibodies that bind PD-L1, CTLA-4, or CD47, etc.), such as pancreatic or prostate cancer, such tumors are referred to as cold tumors. Cancers that respond to treatment with one or more checkpoint inhibitors, such as antibodies that bind PD-L1, CTLA-4, or CD47, etc., such tumors are also referred to as warm or hot tumors. Such tumors are believed to have higher tumor infiltrating lymphocyte (TIL) levels and/or higher tumor mutation loads as compared to tumors that do not respond to checkpoint inhibitor therapy.

The preferred antibody-drug conjugate provided by the present invention also shows extremely significant anti-tumor activity and response rate in a tumor model of a cold tumor and a low-expression PD-L1, and exerts a significant anti-tumor drug effect in various transplantation tumor models.

### Pharmaceutical composition

The present invention also provides a composition. Preferably, the composition is a pharmaceutical composition comprising the antibody above mentioned, or an active fragment thereof, or a fusion protein thereof, and a pharmaceutically acceptable carrier or excipient, and optionally other bioactive substances. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intraperitoneal, intravenous, or topical administration.

The pharmaceutical composition according to the present invention comprises a safe and effective amount (e.g. 0.001-99 wt %, preferably 0.01-90 wt %, preferably 0.1-80 wt %) of the antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. The pharmaceutical composition such as an injection and solution should be manufactured under sterile conditions. The dosage of the active ingredient is a therapeutically effective amount, for example, about 10 µg/kg body weight per day to about 50 mg/kg body weight. In addition, the polypeptide according to the present invention may also be used in combination with an additional therapeutic agent.

When a pharmaceutical composition is used, a safe and effective amount of the immune conjugate is administered to a mammal, wherein the safe and effective amount is typically at least about 10 µg/kg body weight, and in most cases no more than about 50 mg/kg body weight, preferably about 10 µg/kg body weight to about 10 mg/kg body weight. Of course, the specific dosage should also consider factors such as the administration route and the patient's health status, which are all within the skill range of a skilled physician.

### The main advantages of the present invention include:

(a) The present invention provides a combined treatment scheme of the PD-L1 nanobody and the TLR7 agonist for the first time, showing significant *in vivo* anti-tumor activity, indicating that the PD-L1 antibody therapy and the TLR7 immune agonist have a combined rationality and can synergistically resist tumors.
(b) The present invention develops the PD-L1 and TLR7 double-targeting nanobody coupling drug for the first time, which can promote up-regulation expression of PD-L1 in tumors and coordinate innate immunity and adaptive anti-tumor immune responses in tumors, so that the PD-L1 and TLR7 double-targeting nanobody coupling drug show excellent tumor growth inhibition effects in various tumors with poor therapeutic effects of PD-L1 antibodies such as " cold " tumors and low PD-L1 expression.
(c) The PD-L1 and TLR7 double-targeting nanobody coupling drug provided for the first time of the present invention can target a tumor immune microenvironment, reshape a tumor immune microenvironment, improve the infiltration of anti-tumor immune cells, and reduce the infiltration of immunosuppressive cells.
(d) The PD-L1 and TLR7 double-targeting nanobody coupling drug provided for the first time of the present invention exert an explicit anti-tumor drug effect mechanism, and mainly rely on CD8 + T cells and NK cells to exert tumor killing and inhibiting effects.

The present invention will be further illustrated below with reference to the specific examples. It should be understood that these examples are only for illustrating the present invention and not intend to limit the scope of the present invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989, or as instructed by the manufacturers, unless otherwise specified. Unless indicated otherwise, parts and percentage are weight parts and weight percentage.

### Example 1: Anti-PD-L1 nanobody screening

### 1.1. PD-L1 protein expression:

A Mouse PD-L1 (ECD) - pFUSE-hIgG1-Fc vector is constructed and transformed into DH5α chemical competent cells, a monoclonal strain is obtained by screening an LB solid culture medium plate containing bleomycin resistance, and the plasmid is inoculated with bacteria. An HEK293F cell mammal is used to express a PD-L1 protein of a mouse, HEK293F cells are cultured by using a serum-free medium, transfection is completed on the basis of plasmid complexing of PEI, protein supernatant is collected after 5 days of expression of the protein, and protein is purified by means of Protein A affinity column.

### 1.2 Immune animals and library construction

(1) The adult Xinjiang camel is selected, a PD-L1 protein of a 7-time immunized mouse isneeded. A Freund-type complete adjuvant is used for the first time, and a Freund-type incomplete adjuvant is used for subsequent enhanced immunity; (2) after immunization, peripheral blood was extracted from the camel within 3 -5 days, peripheral blood lymphocytes were separated, RNA was extracted by a Trizol method, and reverse transcription was performed to obtain cDNA.; (3) a VHH gene segment was obtained by means of two-step nested PCR amplification.; (4) the VHH gene fragment was connected to the pMECS vector by restriction enzyme PstI and NotI enzyme digestion; (5) the ligation product was electrically converted into *Escherichia coli* electrotransformation competent TG1, and a single-domain antibody library of the mouse PD-L1 protein was constructed; (6) the library was calibrated, and was calculated to obtain the reservoir capacity of 5.2 × 10⁹ CFU by diluting the coating plate; and randomly 24 bacterial colonies were selected by using bacterial colony PCR to detect the library insertion rate. The insertion rate was obtained to be 100% and the above results show that the specific nanobody library of the high-quality targeted mouse PD-L1 was successfully constructed.

### 1.3 Library elutriation

The nanoantibody display phage was obtained by adding auxiliary phage to the 10-fold library of nanoantibody. 96-well enzyme labeling plate was coated with 5 µg/mL of a NeutrAvidin solution (100 µL per well), 4°C, overnight. On the second day, it was closed with 2% skimmed milk powder for 2 h at room temperature, and was washed with 20mM HEPES (pH 7.5), 150 mM NaCl solution for 5 times. The control group and experimental group were set, 100 µL of 200 nM PD-L1 -biotin protein diluent was added, oscillated at room temperature (700 rpm) and incubated for 15 min, and washed with 20 mM HEPES (pH 7.5) and 150 mM NaCl solution for 5 times. Then 100 µl of phage diluent (1 × 10¹³ cfu/mL) was added, oscillated at room temperature (700 rpm) and incubated for 2 h, and the unbound phage was removed by washing with 20 mM HEPES (pH 7.5), 150 mM NaCl solution for 5 times. Then 100 µL of 0.25 mg/mL of pancreatin is added for digestion at room temperature (700 rpm) for 30 min, the specifically bound phage was dissociated, and the inhibitor was added to terminate digestion. The eluted phage is infected with TG1 cells for second round of panning. The above operations are repeated for 2 -3 rounds until the positive clone is enriched.

### 1.4 ELISA Identification of Positive Clone

After several rounds of elutriation, the eluted phage infection is in the TG1 competent state of the growth logarithmic phase, the gradient is diluted and coated on the flat plate to be cultured overnight. 96 clones were picked up and inoculated into a 96-well circular bottom plate of a 100 µL culture medium per well and allowed to stand overnight to serve as a motherboard, and then 10 µL of overnight cultured bacterial liquid was absorbed into a 96-well deep base plate of 1 mL of culture medium per well to induce the expression of the nanobody and crude purification. 96-well enzyme labeling plate was coated with 5 µg/mL of a NeutrAvidin solution, 4°C, 700 rpm, overnight. On the second day, 2% skim milk powder was added to close the plate at room temperature and washed three times with a solution containing bovine serum albumin (BSA). After adding 100 µL 3 µg/mL PD-L1 protein and incubating at room temperature for 30 minutes, the plate was washed. Crude nanobody was added and incubated at room temperature for 1 hour before washing. Mouse anti-HA primary antibody was added and incubated at room temperature for 1 hour before washing. Goat anti mouse alkaline phosphatase labeled secondary antibody was added and incubated at room temperature for 1 hour before washing. Alkaline phosphatase chromogenic solution was added and reacted for 10 minutes. The absorption value was tested at 405 nm on the enzyme analyzer. It is preliminarily determined that it is a positive well, if the absorption value is more than three times the absorption value of the control group., The positive clone was transfered to a shaking tube for culture in order to extract the plasmid and perform sequencing.

### Example 2: Expression and Identification of Anti-PD-L1 Nanobody

After preparing the linearized pFUSE-mIgG2b-Fc and the pFUSE-hIgG1 -Fc vector, the nanobody fragment was amplified by PCR, the nanobody and the vector pFUSE-hIgG1-Fc or pFUSE-m!gG2b-Fc were connected by homologous recombination, then the candidate nanobody was expressed by using the mammalian cell HEK293F, and the nanobody was obtained by purifying the Protein A affinity column.

A preferred nanobody Nb16 was obtained, and the VHH sequence was as shown in SEQ ID NO: 1:
QVQLQESGGGSVQAGGSLRLSCAASGYTDRNYWMGWFRQAPGKEREG VAALYTRTGSTYYADSVKGRFTISHDNAKNTLYLQMNSLKPEDTAVYYCAA DSNAYGLAPLHHYWGQGTQVTVSS
wherein, the underlined mark is shown as CDR part.

In addition, other several nanobodies are obtained in the present invention, which are: Nb9, Nb10, Nb11, Nb17, respectively;
wherein, VHH sequences of Nb9, Nb10, Nb11, Nb17 are respectively as shown in SEQ ID NO: 5, 9, 13 and 17, and CDR part is shown in Table 1.

**Table 1.VHH and CDR sequence of antibody**

| SEQ ID NO. | Name | Sequence |
|---|---|---|
| 1 | Nb16 VHH | QVQLQESGGGSVQAGGSLRLSCAASGYTDRNYWMGWFR QAPGKEREGVAALYTRTGSTYYADSVKGRFTISHDNAKNT LYLQMNSLKPEDTAVYYCAADSNAYGLAPLHHYWGQGT QVTVSS |
| 2 | Nb16 CDR1 | GYTDRNYW |
| 3 | Nb16 CDR2 | LYTRTGST |
| 4 | Nb16 CDR3 | A AD SN A YGL APLHH Y |
| 5 | Nb9 VHH | QVQLQESGGGLVHPGGSLRLSCAASGFTFSSYTMRWVRQA PGKGLEWVSGINNVGGSTLYADSVKGRFTISRDNAKNTLY LQMNSLQPDDTAMYYCATGIIGSGLETATYRGQGTQVTVS S |
| 6 | Nb9 CDR1 | GFTFSSYT |
| 7 | Nb9 CDR2 | INNVGGST |
| 8 | Nb9 CDR3 | ATGIIGSGLETA |
| 9 | Nb10 VHH | QVQLQESGGGSVQAGGSLRLSCAASGYRFCRLAMRWYRQ APGKEREFVSFIDSGGTTSYADSVKGRFTISRDSAKNTVYL QMNSLKPEDTGMYYCNSDILGGGGGCPVSSYWGQGTQVT VSS |
| 10 | Nb10 CDR1 | GYRFCRLA |
| 11 | Nb10 CDR2 | IDSGGTT |
| 12 | Nb10 CDR3 | NSDILGGGGGCPVSSY |
| 13 | Nb11 VHH | QVQLQESGGGSVQAEGSLRLSCSAPIYVTYYGTYCMGWFR QAPGKEREGVAGISGDGRTSYADSVKGRFTVSKDNAKNTL YLEMNSLKPEDTAMYYCALRIGGYCDTLNGADFRYWGQG TQVTVSS |
| 14 | Nb11 CDR1 | IYVTYYGTYC |
| 15 | Nb11 CDR2 | ISGDGRT |
| 16 | Nb11 CDR3 | ALRIGGYCDTLNGADFRY |
| 17 | Nb17 VHH | QVQLQESGGGSVQAGGSLRLSCAASGYSARRRMGWFRQA PGKEREGVASIYTAGGRTWYADSVKGRFTISQDNAKNTVY LQMNSLKPDDTAMYYCAASPAPYGAESLSEADFAYWGQG TQVTVSS |
| 18 | Nb17 CDR1 | GYSARRR |
| 19 | Nb17 CDR2 | IYTAGGR |
| 20 | Nb17 CDR3 | AASPAPYGAESLSEADFAY |

### Example 3: In vitro activity evaluation of anti-human PD-L1 nanobody

### 3.1 PD-L1 binding activity assay of candidate nanobody NB16

Coating the plate with mPD-L1-Fc fusion protein at 4°C overnight, then the plate was sealed with BSA at 37°C for 2 hours, Nb 16 nanobodies of different concentrations were added in each hole, reacted at room temperature for 1 hour, and the plate were washed and goat anti-mouse horseradish peroxidase labeled antibody was added, and reacting at room temperature for 1 hour. After washing, a color developing solution was added, and an absorption value was read at a wavelength of 450 nm.

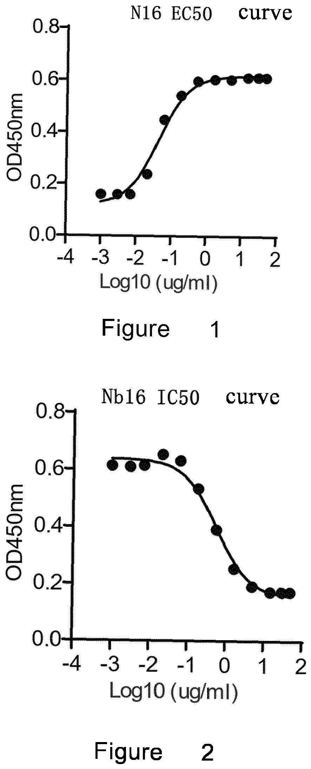

Experimental results show that in this reaction, the EC50 curve of the nanobody Nb16 is shown in FIG. 1, and is 0.045 µg/mL.

### 3.2 PD-1/PD-L1 blocking activity assay of candidate nanobody NB16

Coating the plate with mPD-L1-Fc fusion protein at 4°C overnight, then the plate was sealed with BSA at 37°C for 2 hours, Nb16 nanobodies of different concentrations and 10 µg/mL mouse PD-1-Fc-Biotin fusion protein were added in each hole., After reacting at room temperature for 1 hour, the plate was washed and antibody SA-HRP was added, and the plate reacted at room temperature for 1 hour. After washing, a color developing solution was added, and an absorption value was read at a wavelength of 450 nm.

Experimental results show that, in the reaction, the IC₅₀ curve of the nanobody Nb16 is shown in FIG. 2 as 1.017 µg/mL. The binding of PD-1/PD-L 1 can be effectively blocked.

### 3.3 The in vitro activity of candidate nanobodies NB9, NB10, NB11, NB17

The determination results show that in the candidate nanobodies of the present invention, some of the nanobodies (such as Nb9, Nb10, etc.) are blocking type candidate antibodies, and some of the nanobodies (such as Nb11, Nb17, etc.) are non-blocking nanobodies.

### Example 4: Preparation of TLR7 agonist SZU-101

The molecular structure of TLR7 small molecule agonist for coupling antibody is as follows:

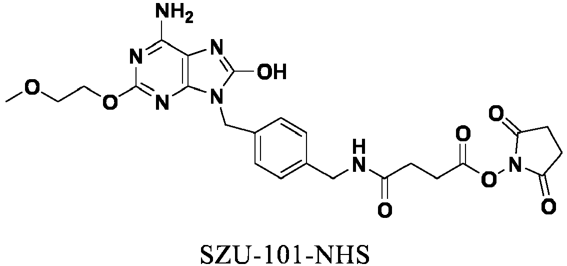

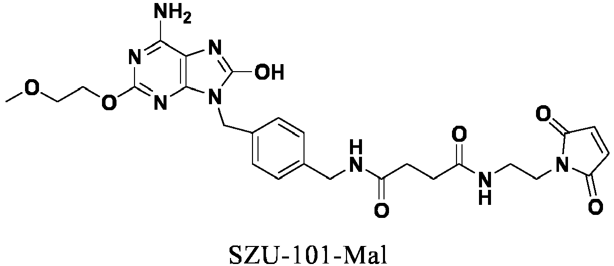

The synthesis of the SZU-101 and the SZU-101 derivative used for coupling may use the following method or similar method.

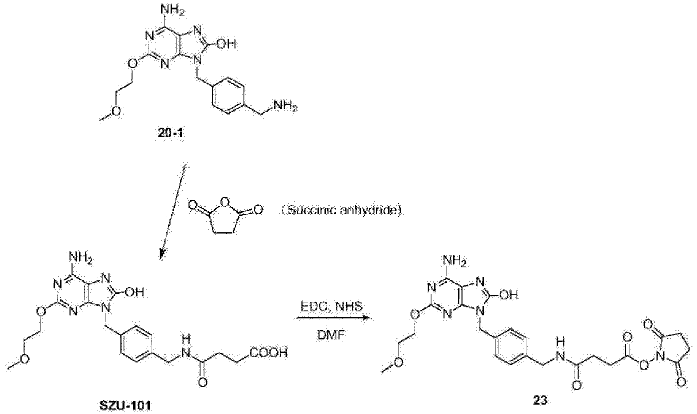

The compound 20-1 was dissolved in anhydrous DMSO, cooled at 10°C to the equivalent amount of succinic anhydride, and the mixture was stirred naturally at room temperature for 24 hours. The mixed reactant was inverted to 20 times the volume of water to precipitate a large amount of white solid compound SZU-101.

The SZU-101 (1 eq), NHS (1.2eq) and EDC (1.3eq) were dissolved in anhydrous DMF, stirred at room temperature for 4 h, the reaction was ended, the reaction solution was poured into dichloromethane, and suction filtration and drying were performed to obtain a compound 23, that is, SZU-101- NHS, which was a white solid.

SZU-101-Mal can be prepared using a similar method.

### Example 5: Combined Anti-Tumor Effect of Nanobody Nb16 and TLR7 Agonist SZU-101

The mouse CT26 tumor cells were recovered and passaged, it was ensured that tumor cells at least have been subjected to 3 generations during tumor-bearing, and mouse IFN-γ with a final concentration of 100 ng/mL was used to induce tumor cells for 24 h before tumor-bearing, so that the cell surface thereof highly expresses PD-L1; the induced high-expression PD-L1 CT26 tumor cell was inoculated subcutaneously into a female BALB/c mouse, wherein the inoculation amount was 1 × 10⁶ cells/one; and randomly grouping mice into four groups (9 per group), i.e., a same-type control group (mFc), a nano-antibody Nb16 group (Nb16), a TLR7 agonist SZU-101 group (SZU-101) and a combined treatment group (NB16/SZU-101), wherein the administration amount of the mFc and the Nb16 was 10 mg/kg, and intraperitoneal administration was performed; and the administration amount of the SZU-101 was 3 mg/kg, and the tumor was administered circumferentially. The administration cycle were Nb16 administered on D1, D5, D9, D12, and D14 and SZU-101 administered on D9-D14 (3A). The body weight of the mouse and the tumor major diameter (L) and the short diameter (W) in the administration cycle were observed and recorded until the anatomical endpoint, and the tumor volume V = (L × W × W)/2 was calculated, a tumor growth curve was drawed, and the tumor inhibition rate was calculated; and the experiment was terminated on the fifteenth day. After performing euthanasia, the animal was dissected, and the subcutaneous tumor was taken out.

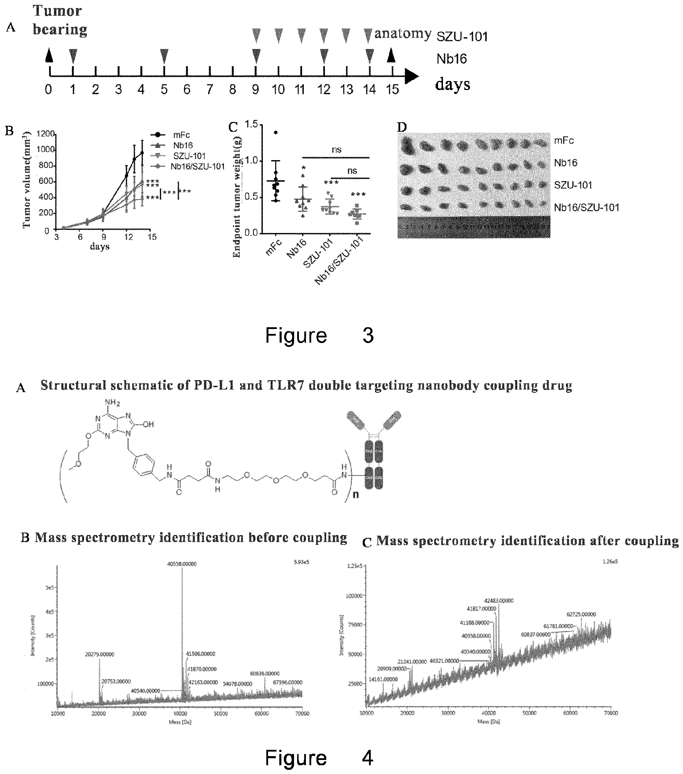

The mouse tumor growth curve (3B), the endpoint tumor weight (3C) and the end tumor anatomical picture (3D) are shown in FIG. 3. The results show that both Nb16 and SZU-101 can significantly inhibit tumor growth, and the combination of both can accidentally exert a more significant anti-tumor effect than single drug treatment, wherein the tumor inhibition rates of the individual drug administration group Nb16 and SZU-101 are respectively 35% and 49%. The tumor inhibition rate of the combined treatment can reach 62%, and the endpoint tumor weight is shown in Table 2.

The above results suggest that the combination of PD-L1 nanobody and TLR7 agonist has a significant synergistic anti-tumor effect.Table 2: Weight of endpoint tumor

| **Group** | **mFc** | **Nb16** | **SZU-101** | **Nb16/ SZU-101** |
|---|---|---|---|---|
| **Endpoint tumor Weight(g)** | 0.755 | 0.381 | 0.516 | 0.245 |
| | 0.458 | 0.664 | 0.286 | 0.391 |
| | 0.671 | 0.361 | 0.328 | 0.255 |
| | 0.845 | 0.418 | 0.426 | 0.301 |
| | 0.533 | 0.806 | 0.298 | 0.295 |
| | 0.589 | 0.505 | 0.560 | 0.265 |
| | 0.686 | 0.460 | 0.330 | 0.391 |
| | 0.633 | 0.450 | 0.278 | 0.230 |
| | 1.398 | 0.250 | 0.354 | 0.146 |
| **Tumor inhibitory rate** | | 35% | 49% | 62% |

### Example 6: Preparation of double-targeted nanobody coupling drug for PD-L1 and TLR7

SZU-101, EDCI and NHS are dissolved in DMSO, stirred at room temperature for 3 h, the LC-MS monitors the reaction process, after the reaction is complete, ten times of double distilled water is added, suction filtration and vacuum drying are performed to obtain the SZU-101-NHS active ester.

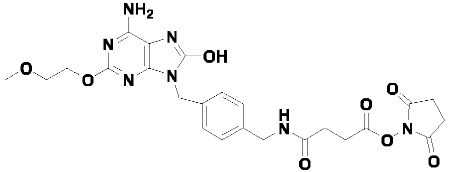

Then, the activated ester is dissolved with DMSO, the antibody and the small molecule react according to a molar ratio of 1: 10, a certain amount of small molecule activated ester is added to Nb16, and reaction is stirred at 4°C for 4 hours. After the reaction is finished, PBS is added to the mixture for mixing, and small molecules are removed by filtering with a 10 KD biological filter membrane to obtain a new coupled compound Nb16-SZU-101. The coupled compound prepared by the reaction first denatures the antibody to open the disulfide bond, and then uses the XevoG2XSQTOF mass spectrometer to identify the sample, and the coupling degree is calculated mainly according to the increased molecular weight of the novel coupling compound than the uncoupled antibody.

As shown in FIG. 4, the coupling degree of the nanobody conjugate obtained by mass spectrometry identification of the nanobody conjugate is 4.5.

By the above method, the PD-L1 and TLR7 dual-targeting nanobody coupling drugs are prepared and named as Nb16 -SZU-101 (FIG. 4A).

### Example 7: In vitro activity assay of PD-L1 and TLR7 dual-targeting nanobody coupling drug

### 7.1 PD-L1 and TLR7 double targeting nanobody coupling drug PD-L1 binding activity assay

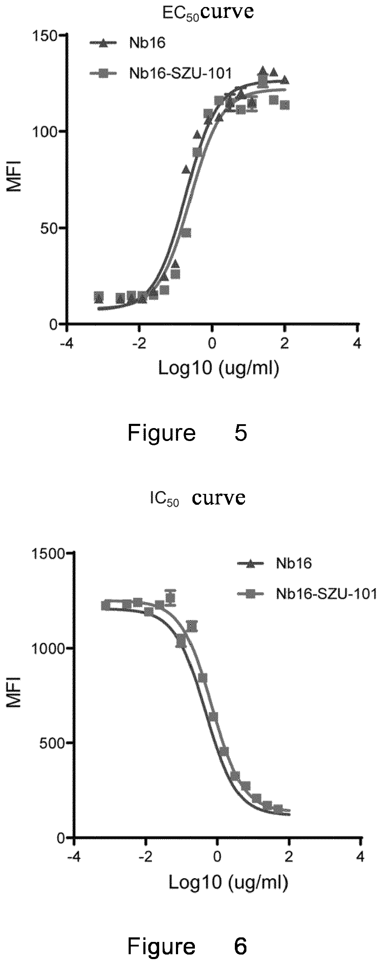

The cell strain HEK293T/mPD-L1 is digested, and centrifugation is performed to remove the supernatant; the cells were washed one time with PBS and the cell density was adjusted to be 2.5 × 10⁶ cells/mL.; each cell sample was added to a 100 µL cell suspension, that is, comprising 2.5 × 10⁵ cell/sample.; different concentrations of coupling compounds Nb16-SZU-101 and bare anti-Nb16 were respectively added into the samples with the concentrations respectively as 100, 50, 25, 12.5, 6.25, 3.12, 1.56, 0.78, 0.39, 0.20, 0.10, 0.05, 0.02, 0.01, 0.006, 0.003, 0.0008 µg/mL, which were 17 concentration gradients; the cells were incubated for 20 min at 4°C; centrifuging, removing supernatant, and the cells were washed once with PBS; using diluted antibody anti-mouse IgG Fc (PE) as secondary antibody, and the cells were re-suspended; incubated at 4°C for 20 min; centrifuged, supernatant was removed, and the cells were washed twice with PBS, and cells were transferred to a flow tube; and were performed on-machine detection by using a flow cytometer to obtain an EC 50 value curve of Nb16-SZU-101, as shown in FIG. 5.

Experimental results show that the coupling of SZU-101 does not change the binding activity of the original PD-L1 nanobody.

### 7.2 PD-L1 and TLR7 double targeting nanobody coupling drug PD-1/PD-L1 blocking activity assay

The cell strain HEK293T/mPD-L1 was digested, and centrifugation was performed to remove the supernatant; washing one time with PBS and adjusting the cell density to be 2.5 × 10⁶ cells/mL; each cell sample was added to a 100 µL cell suspension, that is, comprising 2.5 × 10⁵ cell/sample; mouse PD-1-biotin protein (20µg/mL ) and different concentrations of coupling compounds Nb16-SZU-101 and bare anti-Nb16 were respectively added into the samples with the concentrations respectively as 100, 50, 25, 12.5, 6.25, 3.12, 1.56, 0.78, 0.39, 0.20, 0.10, 0.05, 0.02, 0.01, 0.006, 0.003, 0.0008 µg/mL, which were 17 concentration gradients; the cells were incubated for 20 min at 4°C; centrifuging, removing supernatant, and the cells were washed once with PBS; using diluted antibody SA-PE as secondary antibody, and the cells were re-suspended; incubated at 4°C for 20 min; centrifuge, supernatant was removed, and the cells were washed twice with PBS, and cells were transferred to a flow tube; and were performed on-machine detection by using a flow cytometer to obtain an IC50 curve of Nb16-SZU-101, as shown in FIG. 6.

Experimental results show that the coupling of SZU-101 does not change the blocking activity of the original PD-L1 nanobody.

### Example 8: Evaluation of antitumor activity of PD-L1 and TLR7 double-targeted nanobody cooupling drugs in vivo

### 8.1 In vivo anti-tumor effects of double targeting nanobody coupling compound and combined administration group

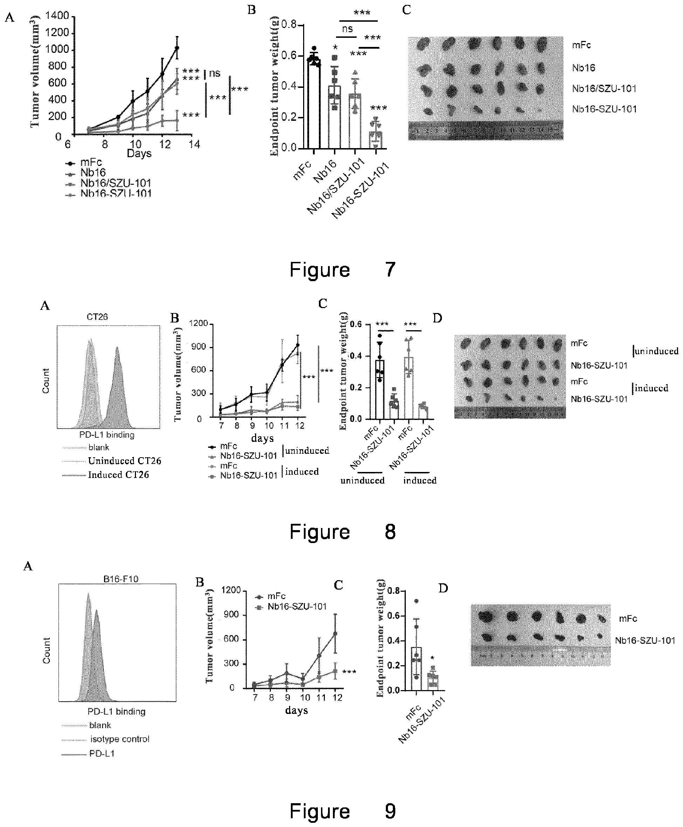

In an IFN-γ-induced high-expression PD-L1 CT26 subcutaneous tumor-bearing BALB/c mouse model, mice were randomly divided into four groups, namely an mFc group, an Nb16 group, an Nb16/SZU-101 combined administration group and an NB16 -SZU-101 nanobody conjugate drug group, wherein the administration dosage of the mFc or Nb 16 or Nb16-SZU-101 was 10 mg/kg, the administration mode was intraperitoneal administration, the administration dosage of the SZU-101 was 0.5 mg/kg, and the administration mode was intraperitoneal administration; the drug was administered on Day 2, Day 6, Day 10 and Day 13 for a total of 4 times. Tumor sizes were measured 2 -3 times per week, and tumor growth curves were drawn; and anatomical weighing and photographing were performed on mice at Day 14. The tumor growth curve, the endpoint tumor weight and the endpoint tumor photowere shown in FIG. 7.

Experimental results show that the nanobody coupling drug Nb16-SZU-101 treatment group of the present invention has a significantly improved anti-tumor effect. In the model, the tumor inhibition rate of the individual drug administration group Nb16 is 30%, the tumor inhibition rate of the combined administration group Nb16/SZU-101 is 39%, the tumor inhibition rate of the nanobody coupling drug group can reach 81%, and the endpoint tumor weight is shown in Table 3. The nanobody coupling drug group can significantly inhibit tumor growth regardless of whether the group is administered alone or with respect to the combined administration group.

It should be noted that, compared with a single administration group Nb16, the combined administration group does not significantly inhibit tumor growth, which may be due to the reduction of the dose of SZU-101 in the combined administration group and consistent with the coupling group, the administration mode is changed into abdominal cavity administration, and the administration utilization degree of the abdominal cavity administration relative to the tumor circumference is lower.

**Table3: Weight of endpoint tumor**

| **Group** | **mFc** | **Nb16** | **Nb16/ SZU-101** | **Nb 16-SZU-101** |
|---|---|---|---|---|
| **Endpoint tumor Weight(g)** | 0.543 | 0.342 | 0.280 | 0.155 |
| | 0.553 | 0.334 | 0.340 | 0.084 |
| | 0.595 | 0.494 | 0.434 | 0.012 |
| | 0.585 | 0.442 | 0.244 | 0.087 |
| | 0.576 | 0.263 | 0.496 | 0.191 |
| | 0.652 | 0.591 | 0.343 | 0.146 |
| **Tumor inhibitory rate** | | 30% | 39% | 81% |

### 8.2 Uninduced (low expression) PD-L1 and induced (high expression) PD-L1 CT26 subcutaneous tumor-bearing BALB/c mouse model

In FIG. 8A, it has been shown that the non-induced CT26 cell surface PD-L1 expression is approximately 17.3%, and the expression level of PD-L1 is increased to 98.1% only after IFN-γ induction. Therefore, we researched and compared the effects of the coupling compound in the non-induced and induced CT26 mouse tumor model, respectively.

In the two models, CT26 tumor cells were subcutaneously inoculated into female BALB/c mice before and after induction respectively to construct tumor-bearing mouse models, and the inoculation amount was 1 × 10⁶ cells/one; the mice are randomly divided into two groupswith 6 mice of each group, that is, a blank control group mFc (the concentration is 10 mg/kg), and a coupling compound Nb16-SZU-101 group (the concentration was 10 mg/kg); the administration dosage was 200 µl, and the administration modes were both intraperitoneal administration; administration was performed on Day 2, Day 5, Day 8 and Day 11 respectively, and administration was carried out for 4 times; the size of the tumor was measured for 2-3 times every week, and a tumor growth curve was drawn; and anatomical weighing and photographing were performed on the mouse at Day 12.

Results of the tumor growth curve showed that in two tumor models before and after CT26 induction, the coupling compound can significantly inhibit tumor growth in two tumor models before and after CT26 induction (FIG. 8B), wherein the inhibition rate in the non-induced CT26 model is 78.9%, the inhibition rate in the induced CT26 model is 83.6%, and the tumor inhibition effect of the coupling compound in the two models is not obviously different. In addition, the results of the endpoint tumor weight (FIG. 8C) and the endpoint tumor photo (FIG. 8D) are also basically consistent with the endpoint tumor volume result. It is confirmed that the coupling compound Nb16-SZU-101 exerts a significant inhibitory effect on both PD-L1 low expression and highly expressed tumors.

This indicates that the PD-L1 and TLR7 double-targeting nanobody coupling drug provided by the present invention not only has a significant anti-tumor effect on " hot " tumors with high expression of PD-L1, but also can be used for anti-tumor treatment of PD-L1 low-expression " cold " tumors.

### 8.3 Uninduced B16 -F10 subcutaneous tumor-bearing C57BL/6 model with low expression PD-L1

On the basis of the inhibitory effect of the PD-L1 and TLR7 double-targeting nanobody coupling drug on the CT26 tumor with low expression of PD-L1, the present invention further evaluated the effect of the PD-L1 and TLR7 dual-targeting nanobody coupling drug in a low-expression PD-L1 C57BL 6 mouse B16F10 subcutaneous tumor-bearing model.

The 6 to 8 - week-old C57BL 6 mice were randomly divided into two groups, which were respectively as an mFc group and a nanobody-coupled drug NB 16 - SZU-101 group. 1 × 10⁶ B16 -F10 cells (FIG. 9A) of 1 × 10⁶ non-induced low-expression PD-L1 were planted under the underarm skin of mice (FIG. 9A). Administration was performed by using mFc and nanobody coupled drug Nb16 - SZU-101, respectively; the administration dosage of the control drug and Nb16-SZU-101 was 10 mg/kg, and the volume is 200 µL; each group of drug delivery manners was intraperitoneal injection. Each group was administered at 2th, 5th, 8th and 11th days after tumor implantation, respectively, and the drug was administered for 5 times. On Day 12 mice would be euthanized. The tumor growth curve, the endpoint tumor weight and the endpoint tumor photo were shown in FIGs. 9B-D.

Experimental results show that in the tumor model of the low-expression PD-L1, the nanobody-coupled drug Nb 16 - SZU-101 treatment group has a significantly improved anti-tumor effect, and in the model, the tumor inhibition rate is about 68.2%, which prompts that the PD-L1 and TLR7 double-targeting nanobody coupling drug provided by the present invention can be used for anti-tumor treatment of PD-L1 low-expression tumors.

A therapeutic drug or solution targeting PD-L1 is usually only a tumor highly expressed for PD-L1, but cannot be effectively used for PD-L1 low-expression or moderate-expression tumors. Therefore, the double-target nanobody coupling drug of the present invention may be used to treat tumors with low expression or moderate-expression of PD-L1, which is unexpected.

### 8.4 early/late CT 26 subcutaneous tumor-bearing BALB/c mouse model

Based on the superior anti-tumor drug effect of the dual-target nanobody coupling drug, we further evaluated the tumor inhibition effect thereof in the CT26 early model (tumor volume < 50 mm³) and late model (tumor volume > 200 mm³).

The uninduced CT26 tumor cell subcutaneous was inoculated into a female BALB/C mouse to construct a tumor-bearing mouse model in the two models, and the inoculation amount was 1 × 10⁶ cells/; the mice were divided into two groups, and each group was 8 mice, that is, a blank control group mFc (the concentration was 10 mg/kg), and a coupling compound Nb16 - SZU-101 group (the concentration was 10 mg/kg); and the administration dosage was 200 µL, and the administration manner was an abdominal administration. In the early model (the tumor volume was < 50 mm³), the drug was administered in Day 4, Day 7, Day 10, Day 13, Day 16, and the drug was administered for 5 times; in the late model (the tumor volume was greater than 200 mm³), the drug was administered in Day 9, Day 12, Day 15, Day 17, and the drug was administered for 4 times. The tumor size was measured 2 -3 times a week, and a tumor growth curve was drawn.

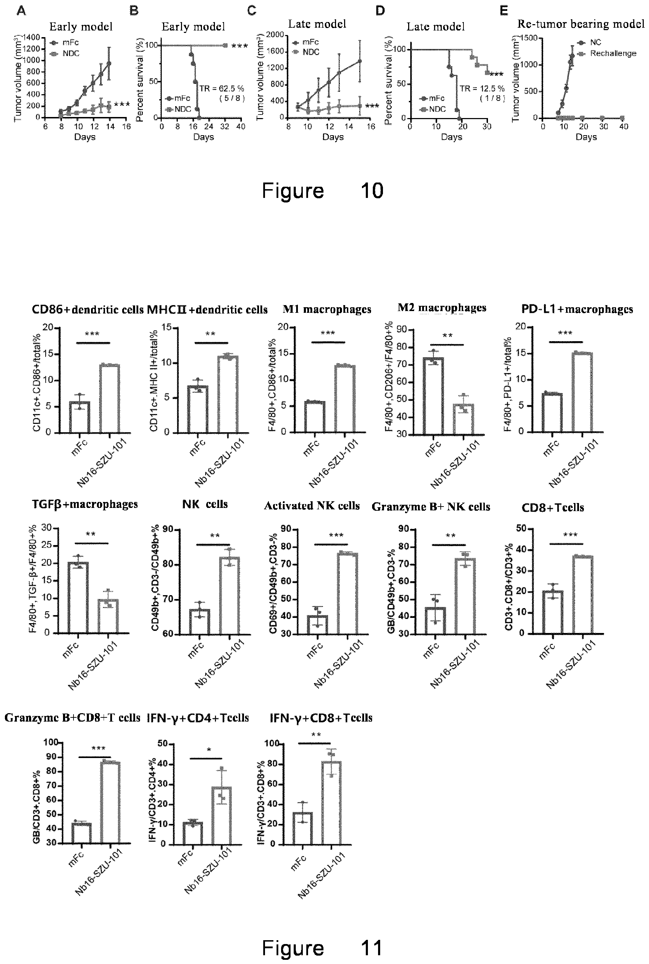

Results of the tumor growth curve show that in two tumor models of the early/late CT26, the coupling compound can significantly inhibit tumor growth, wherein the inhibition rate in the early model is 80.3% (FIG. 10A), and the inhibition rate in the late model is 78.9% (FIG. 10C); in addition, in the two models, the double-target nanobody coupling compound can significantly prolong the lifetime (FIG. 10B and FIG. 10D). Moreover, in the early model, the coupling compound therapy will cause tumor regression of up to 62.5% tumor-bearing mice (FIG. 10B); in the late model, the compound drug will cause 12.5% tumor-bearing mice to have tumor regression (FIG. 10D). After the tumor of the mouse fades for 1 week, re-tumor of CT26 tumor cells is performed on the same, and the mouse does not grow the tumor within 40 days (FIG. 10E), indicating that the coupling compound induces an effective anti-tumor immune memory, and the above result is a crying.

### Example 9: Tumor immunotyping analysis of PD-L1 and TLR7 double-targeted nanobody coupling drug administration

At a mouse anatomical endpoint of example 8.1, subcutaneous tumors were taken, 150 mg of tumor tissue was taken and cut into meat paste, and the meat paste was oscillated and digested at 180 rpm for 1.5 h at 37°C in hyaluronidase and collagenase IV, and processed into a single cell suspension. The pretreated cell suspension was divided into two parts, one part was used for immunotyping analysis of macrophages and dendritic cells, and one part was used for immune typing analysis of T cells and NK cells.

The suspension treatment of macrophages and dendritic cell samples: the cells were treated with 2 -4 mL sterile red blood cell lysis solution for 8 min and terminated with PBS buffer solution to remove red blood cells and fragments in the sample suspension; the cell sample was cleaned for use.

T cell and NK cell sample suspension treatment: the cells were centrifuged with lymphocyte separation liquid to obtain a lymphocyte separation layer, that is, the lymphocyte separation layer was a tumor lymphocyte suspension, and wascleaned for use.

The macrophages and dendritic cell samples and T cells and NK cell samples were sealed at 4°C with an Fc receptor confining liquid for 1 hour, and non-specific staining caused by the Fc receptor was removed; cell surface protein staining is performed, specifically: M1 macrophages (FITC Rat Anti-Mouse CD45, PE-Cyanine7 Rat Anti-Mouse F4/80 and APC Rat anti-Mouse CD86), M2 macrophages (FITC RATw Anti-Mouse CD45 and PE-Cynine 7 RAT Anti-Mouse F4/80), dendritic cells (PE-Cy7 Anti-Mouse CD11c, BB515 Rat Anti-Mouse I-A/I-E, PE Hamster Anti-Mouse CD80, and APC Rat anti-Mouse CD86), CD4 + T cells and CD8 + T cells (FITC Hamster Anti-Mouse CD3e, PerCP-Cy ^{™} 5.5 Rat Anti-Mouse CD4 and APC Rat Anti-Mouse CD8a), Treg cells (FITC Hamster Anti-Mouse CD3e, PerCP-CYy^{™} 5.5 Rat Anti-Mouse CD4 and PE Rat Anti-Mouse CD25), NK cells (CD3 -FITC, CD49b-APC, CD69 -PE), followed by dyeing on ice for 20 min.; then intracellular protein staining is performed, and after the cell is subjected to a fixed membrane rupture treatment, the cell is subjected to membrane rupture dyeing, specifically: M2 macrophages (MS CD206 Alexa 647 and ANTI-MOUSE LAP PE), functional T cells(ANTI-MOUSE GRANZYME B (NGZB) PE) containing granzyme, functional T cells (PE Rat Anti-Mouse IFN-γ) containing IFN-γ, Treg cells (ANTI-MOUSE/RAT FOXP3 APC), and then stained for 20 min on ice; after cleaning, re-suspending the sample cell sap, and performing on-machine detection on the flow cytometer.

The flow-type immune typing result was shown in FIG. 11.

As a result, PD-L1 and TLR7 double-targeting nanobody coupling drug Nb16-SZU-101, compared to the same-type control mFc group, promotes maturation of dendritic cells. Meanwhile, the PD-L1 and TLR7 double-targeting nanobody coupling drug significantly increases the function of tumor-infiltrating cytotoxic cells (CD8 + T cells and NK cells), and promote the expression of their granzyme B and IFN-γ. In addition, the PD-L1 and TLR7 double-targeting nanobody coupling administration can promote the re-polarization of tumor-associated macrophages and reduce the infiltration of TGF-β+ macrophages. In the Nb16 - SZU-101 treatment group, the infiltration of IFN-γ + CD4 + T cells is also significantly increased.

Surprisingly, the drug delivery of PD-L1 and TLR7 double-targeting nanobody coupling drug promotes macrophage expression of PD-L1 (FIG. 11) in a tumor microenvironment, which can enable PD-L1 and TLR7 double-targeting nanobody coupling drug to respond to " cold " tumors with low expression of PD-L1 molecules, achieving a wider response, and having a better anti-tumor drug effect. In addition, in a tumor microenvironment, the immune cells (including CD4 + T cells and CD8 + T cells, etc.) activated by the conjugate of the present invention increase the immune cell subtype of IFN-γ, while IFN-γ is one of the most common cytokines for inducing PD-L1 expression, and therefore, the expression of PD-L1 by tumor cells can be further promoted, so that the " cold " tumor is converted into a " hot " tumor.

As described above, the PD-L1 and TLR7 double-targeting nanobody coupling drug targets tumor immune microenvironment, and can reshape tumor immune microenvironment, coordinate innate immunity and adaptive immunity to exert anti-tumor activity.

### Example 10: Immune cell deletion analysis to determine the immune basis for the efficacy of PD-L1 and TLR7 double-targeted nanobody coupling drug

Balb/c mice were randomized into 6 groups of 6 mouse each. Corresponding groups are: MFC group, Nb16-SZU-101 group, Nbl6-SZU-101 + anti-CD4 group, Nb16-SZU-101 + anti-CD8 group, Nb16-SZU-101 + anti-NK1.1 group, Nb16-SZU-101 + chlorophosphate liposome group. The administration of the mFc and Nb16-SZU-101 was consistent with example 8.1.

The immune cell deletion method comprised: deleting corresponding immune cells (CD4 + T cells, CD8 + T cells, NK cells) by intraperitoneal injection of antibody drugs (anti-CD4, anti-CD8, anti-NK1.1) respectively in Day 3 (200 ug/one) / Day 4 (100 ug/one) / Day 5 (100 ug/one), or intraperitoneal injection of chlorophosphate-liposomes to remove macrophages.

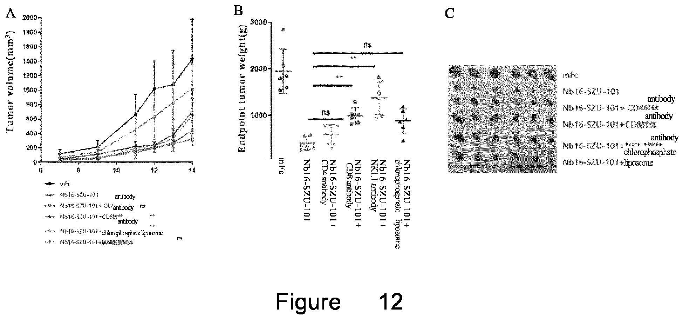

On Day 14 the mice would be euthanized. The tumor growth curve and the endpoint tumor photo were shown in FIG. 12.

The results show that the deletion of CD8 + T cells and NK cells greatly affects the efficacy of PD-L1 and TLR7 double-targeting nanobody coupling drugs, while the deletion of CD4 + T cells and macrophages has little influence on the efficacy of PD-L1 and TLR7 double-targeting nanobody coupling drugs, which indicates that the *in vivo* anti-tumor activity of PD-L1 and TLR7 double-targeting nanobody coupling drugs provided by the present invention mainly functions by CD8 + T cells and NK cells, and the killer cells may not be replaced in drug efficacy.

### Example 11: Screening and expression of Anti-human PD-L1 nanobody

As described in example 1, after the human PD-L1 protein was expressed and immunized with a camel, a library was constructed and library elutriationwas performed, and 11 candidate positive clones were obtained by ELISA identification.

As described in example 1, the candidate nanobody sequences were homologous and recombined into the pFUSE-mIgG2b-Fc and the pFUSE-hIgG1-Fc vector, and then the candidate nanobodies were expressed by using the mammalian cells HEK293F.

In the present invention, 11 strains preferred anti-human PD-L1 nanobodies were obtained, which were respectively h_Nb1, h_Nb2, h_Nb4,h_Nb5, h_Nb6, h_Nb9, h_Nb12, h_Nb13, h_Nb19, h_Nb26, h_Nb30;
wherein the VHH sequences of h_Nb1, h_Nb2, h_Nb4, h_Nb5, h_Nb6, h_Nb9, h_Nb12, h_Nb13, h_Nb19, h_Nb26, h_Nb30 are shown in SEQ ID NO: 21, 25, 29, 33, 37, 40, 44, 48, 52, 56, 59, respectively, and CDR parts are shown in Table 4.

**Table4: VHH and CDR sequence of anti-human PD-L1 abtibody**

| SEQ ID NO. | Name | Sequence |
|---|---|---|
| 21 | h_Nb1 VHH | DVQLQESGGGLVQPGGSLRLSCAASGFTFSSYAMSWVRQA PGKGLEWVSDINSGGDNTDYADSVKGRFTISRDNAKNTLY LQMNSLKPEDTAVYYCARGPKLCFSSWGQGTQVTVSS |
| 22 | h_Nb1CD R1 | GFTFSSYA |
| 23 | h_Nb1 CDR2 | INSGGDNT |
| 24 | h_Nb1 CDR3 | ARGPKLCFSS |
| 25 | h_Nb2 VHH | DVQLQESGGGLVQPGGSLRLSCAASGFTFGDRWMYWLRQ APGKGLEWVSSINPAGRSTYYADSVKGRFTISRDNAKNTL YLQMNSLKSEDTAVYYCTKDPGGYQKGQGTQVTVSS |
| 26 | h_Nb2 CDR1 | GFTFGDRW |
| 27 | h_Nb2 CDR2 | INPAGRST |
| 28 | h_Nb2 CDR3 | TKDPGGYQ |
| 29 | h_Nb4 VHH | DVQLQESGGGLVQAGGSLRLSCAAPSRMFSGYTMGWYRQ APGKQRELVAQIANGGFTDYIDSVKGRFSISRDTVLNMMY LQMNSLKPEDTAVYYCNLVLVNGIPTRNWGQGTQVTVSS |
| 30 | h_Nb4 CDR1 | SRMFSGYT |
| 31 | h_Nb4 CDR2 | IANGGFT |
| 32 | h_Nb4 CDR3 | NLVLVNGIPTRN |
| 33 | h_Nb5 VHH | DVQLQESGGGAVQSGESLRLSCAASGRDLMFYDVAWFRQ APGKEREGVSYIRSSGTLTKYADSVKGRFTISRDNDKNTVS LQMNSLNPEDTAKYYCAARLQSATSHSDLREDEFNSWGQ GTQVTVSS |
| 34 | h_Nb5 CDR1 | GRDLMFYD |
| 35 | h_Nb5 CDR2 | IRSSGTLT |
| 36 | h_Nb5 CDR3 | AARLQSATSHSDLREDEFNS |
| 37 | h_Nb6 VHH | DVQLQESGGGLAQPGGSLRLSCAASGFTFSSYAMSWARQA PGKGLEWVAGIYRGGSDTYYADSVKGRFTISRDNTKNTLY LQMNSLKPEDTALYYCIQGWGLGYANYDYWGQGTQVTV SS |
| 22 | h_Nb6 CDR1 | GFTFSSYA |
| 38 | h_Nb6 CDR2 | IYRGGSDT |
| 39 | h_Nb6 CDR3 | IQGWGLGYANYDY |
| 40 | h_Nb9 VHH | DVQLQESGGGLVQPGGSLRLSCASSGSIFGLGVKGWYRQP PGNQREMVAQIISGGRISYADSVKGRFTISRDNAKNTVYLQ MNSLKPEDTAVYYCNAWGPGQRNYWGRGTQVTVSS |
| 41 | h_Nb9 CDR1 | GSIFGLGV |
| 42 | h_Nb9 CDR2 | IISGGRI |
| 43 | h_Nb9 CDR3 | NAWGPGQRNY |
| 44 | h_Nb12 VHH | DVQLQESGGGLVQAGGSLRLSCAASGRTFSSRAMGWFRQ APGKEREFVAAISGSGRNTYYADSVKGRFTISRDNAKNSV NLQMNSLKPEDTAVYYCNAAGGITIATMTQRTQYDYWG QGTQVTVSS |
| 45 | h_Nb12C DR1 | GRTFSSRA |
| 46 | h_Nb12 CDR2 | ISGSGRNT |
| 47 | h_Nb12 CDR3 | NAAGGGITIATMTQRTQYDY |
| 48 | h_Nb13 VHH | DVQLQESGGGLVQPGGSLRLSCAASGSIFSINAMGWYRQA PGKSRELVAAITSGGSTNYADSVKGRFTISRDTAKNTVYLL MNSLEPEDTAVYYCNVERRRPPHYLSEYDFDWGQGTQVT VSS |
| 49 | h_Nb13C DR1 | GSIFSINA |
| 50 | h_Nb13 CDR2 | ITSGGST |
| 51 | h_Nb13 CDR3 | NVERRRPPHYLSEYDFD |
| 52 | h_Nb19 VHH | DVQLQESGGGLVQAGGSLRLSCAASGRTFSGYAMAWFRQ APGKEREFVAAISGSGGRTYYADSRFTISRDNAKNTLYLQ MNSLKPEDTAGYYCAAAGEGITIATMTQRTQYDYWGQGT QVTVSS |
| 53 | h_Nb19C DR1 | GRTFSGYA |
| 54 | h_Nb19 CDR2 | ISGSGGRT |
| 55 | h_Nb19 CDR3 | AAAGEGITIATMTQRTQYDY |
| 56 | h_Nb26 VHH | DVQLQESGGGLVQPGGSLRLSCAAPGSIFRINAMGWYRQA PGKQRELVAAITSGGSTNYADSVQGRFTISRDNAKNTVYL QMNSLKPEDTAVYYCNVERRRPPHYYSDYDFDWGQGTQV TVSS |
| 57 | h_Nb26C DR1 | GSIFRINA |
| 50 | h_Nb26 CDR2 | ITSGGST |
| 58 | h_Nb26 CDR3 | NVERRRPPHYYSDYDFD |
| 59 | h_Nb30 VHH | DVQLQESGGGLVQPGESLRVSCAASGFTFSNYYMTWVRQ APGKGLEWVSFLNPSSSERFYADSVKGRFTISRDNAKNTLD LLMHSLKPEDTALYYCARHLKYNWAPNYDYWGQGTQVT |
| | | VSS |
| 60 | h_Nb30C DR1 | GFTFSNYY |
| 61 | h_Nb30 CDR2 | LNPSSSER |
| 62 | h_Nb30 CDR3 | ARHLKYNWAPNYDY |

### Example 12: Preliminary in vitro activity evaluation of anti-human PD-L1 nanobody

### 12.1 Human PD-L1 binding activity assay of candidate anti-human PD-L1 nanobody

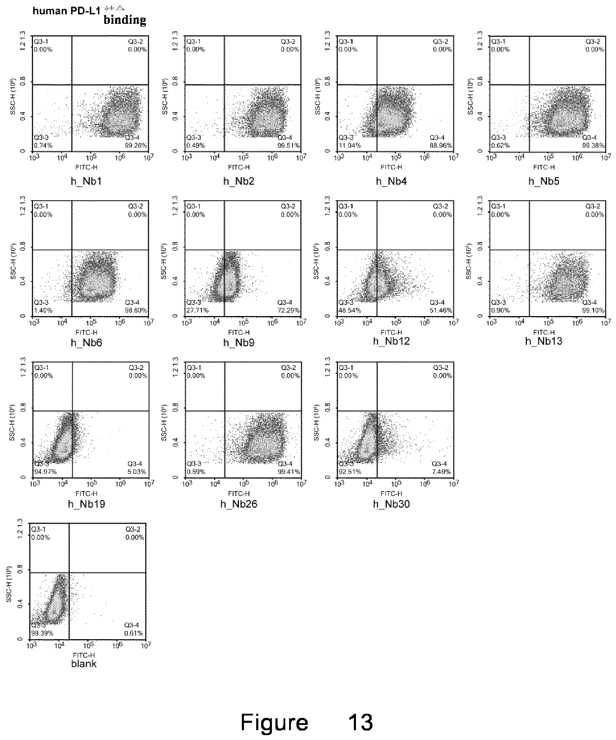

The cell strain HEK293T/hPD-L1 was digested, and centrifugationwas performed to remove the supernatant; the cells were washed one time with PBS and the cell density was adjusted to 2.5 × 10⁶ cells/mL; each cell sample was added to a 100 µL cell suspension, that is, a 2.5 × 10⁵ cell/sample was comprised; different anti-human PD-L1 candidate nanobodies (the concentration is 10 µg/mL) were respectively added to the samples; incubating for 20 min at 4°C; centrifuging, removing supernatant, and the cells were washing once with PBS; using diluted antibody anti-human IgG Fc (FITC) as secondary antibody, and the cells were re-suspended; incubating at 4°C for 20 min; centrifuging, removing supernatant, the cells were washing twice with PBS, and transferred to a flow tube; and was performed on-machine detection by using a flow cytometer to obtain the binding activity of the candidate nanobody and human PD-L1, as shown in FIG. 13.

Experimental results indicate that the candidate nanobodies h_Nb1, h_Nb2, h_Nb4, h_Nb5, h_Nb6, h_Nb9, h_Nb12, h_Nb13, h_Nb26 total 9 nanobodies maintain good binding activity with human PD-L1.

### 12.2 Human PD-1/PD-L1 blocking activity assay of candidate anti-human PD-L1 nanobody

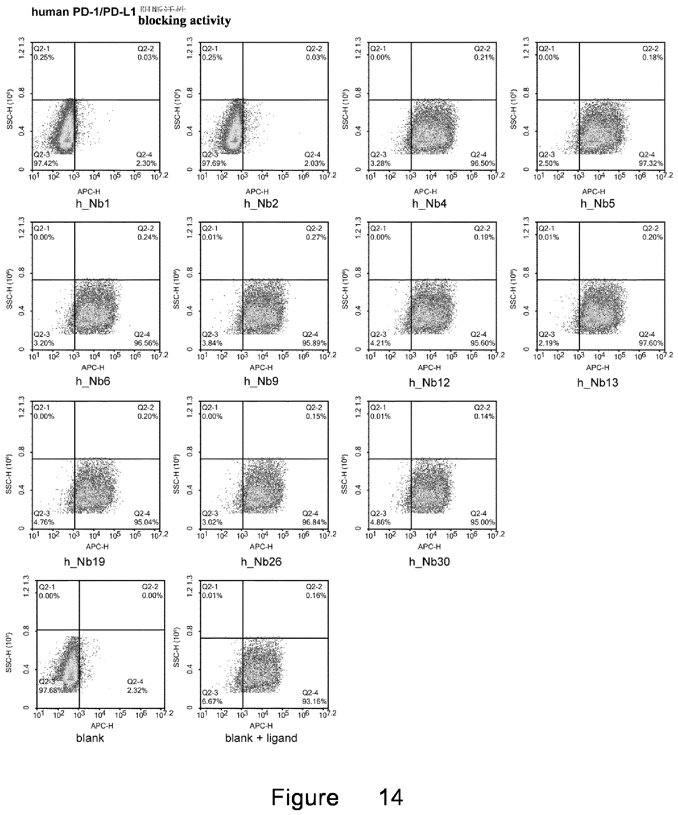

The cell strain HEK293T/hPD-L1 was digested, and centrifugationwas performed to remove the supernatant; the cells were washed one time with PBS and the cell density was adjusted to 2.5 × 10⁶ cells/mL; each cell sample was added to a 100 µL cell suspension, that is, a 2.5 × 10⁵ cell/sample was comprised; and the human PD-1-his protein (the concentration was 1 µg/mL) and the anti-human PD-L1 candidate nanobody (the concentration was 10 µg/mL) were respectively added to the sample; the cells were incubated for 20 min at 4°C; centrifuging, supernatant was removed, and the cells were washed once with PBS; using diluted antibody anti-his-APC as secondary antibody, and the cells were re-suspended; incubated at 4°C for 20 min; centrifuging, supernatant was removed, the cells were washedtwice with PBS, and was transferred to a flow tube; and was performed on-machine detection by using a flow cytometer to obtain the PD-1/PD-L1 blocking activity of the candidate nanobody, as shown in FIG. 14.

Experimental results show that the candidate nanobodies h _ Nb1 and h_Nb2 can effectively block the binding activity of PD-1/PD-L 1, and have an obvious blocking effect.

### Example 13: In vitro activity evaluation of anti-human PD-L1 nanobody

### 13.1 EC₅₀ activity assay of candidate anti-human PD-L1 binding with human PD-L1

As described in example 12.1, the cell strain HEK293T/hPD-L1 was digested and was treated to prepares a cell sample; nanobodies h_Nb1 or h_Nb2 or positive control antibody KN035 with different concentrations were respectively added to the sample; after incubation centrifugation was finished, anti-human IgG Fc (FITC) was used as secondary antibody, and the cells were incubated and stained; and then on-machine detection was performed by using a flow cytometer to obtain an EC₅₀ value combining the candidate nanobody with human PD-L1.

### 13.2 Nanobody of candidate anti-human PD-L1 blocks human PD-1/PD-L1 combined IC₅₀ activity assay

As described in Example 12.2, the cell strain HEK293T/hPD-L1 was digested and was treated to prepares a cell sample; nanobody h_Nb1 or h_Nb2 or positive control antibody KN035 having different concentrations was added respectively, and human PD-1-his protein (with a concentration of 1 µg/m); after the incubation centrifugation was finished, using anti-his-APC as a secondary antibody, and the cells were incubated and stained; and then performing on-machine detection by using a flow cytometer to obtain an IC₅₀ value of the candidate nanobody blocking human PD-1/PD-L1 binding.

### Example 14: Evaluation of antitumor activity of anti-human PD-L1 and TLR7 double-targeted nanobody coupling drug in vivo

As described in Example 6, anti-human PD-L1 and TLR7 double-targeting nanobody coupling drugs h_Nb1-SZU-101 and h_ Nb2-SZU-101 were prepared.

In order to further evaluate the *in vivo* anti-tumor activity of the anti-human PD-L1 and TLR7 double-targeting nanobody coupling drug, we used a CT26/hPD-L1 tumor model established by a PD-1/PD-L1 anthropomorphic BALB/c mouse, wherein the administration dosage was 10 mg/kg, and the administration mode was intraperitoneal administration. The tumor size was measured 2 -3 times a week, and a tumor growth curve was drawn.

### Example 15: Humanized of anti-human PD-L1 nanobody

The human antibody FR was replaced with a camel antibody FR by using a CDR Grazing method, so as to achieve the purpose of reducing immunogenicity. Firstly, candidate antibodies were performing homologous modeling, identified to obtain key amino acid residue sites, performed homologous structure search in a structure database by taking the candidate nanobody sequences as templates, selected an optimal structure sequence for sequence replacement to finally obtain a humanized antibody sequence, and was considered the potential influence of the reserved framework region on the key sites under the CDR effect.

In the present invention, the obtained 11 preferred anti-human PD-L1 nanobodies were humanized respectively, wherein the corresponding sequence after the humanized antibody is shown as follows:
The humanized h_Nb1 sequences were h_Nb1_1, h_Nbl_2, h_Nb1_3, h_Nb1_4 and h_Nbl_5, respectively;
The humanized h_Nb2 sequences were h_Nb2_1, h_Nb2_2, h_Nb2_3, h_Nb2_4 and h_Nb2_5, respectively;
The humanized h_Nb4 sequences were h_Nb4_1, h_Nb4_2, respectively;
The humanized h_Nb5 sequences were h_Nb5_1, h_Nb5_2, h_Nb5_3 respectively;
The humanized h_Nb6 sequences were h_Nb6_1, h_Nb6_2, respectively;
The humanized h_Nb9 sequences were h_Nb9_1, h_Nb9_2, respectively;
The humanized h_Nb12 sequences were h_Nb12_1, h_Nb12_2, respectively;
The humanized h_Nb13 sequences were h_Nb13_1, h_Nb13_2, respectively;
The humanized h_Nb19 sequences were h_Nb19_1, h_Nb19_2, respectively;
The humanized h_Nb26 sequences were h_Nb26_1, h_Nb26_2, respectively;
The humanized h_Nb30 sequences were h_Nb30_1, h_Nb30_2, respectively;
The humanized antibody sequences are shown in Table 5.

**Table5: Humanized VHH sequence of anti-human PD-L1 abtibody**

| SEQ ID NO. | Name | Sequence |
|---|---|---|
| 63 | h_Nb1_1 | QVQLVESGGGLVQPGGSLRLSCAASGFTFSSYAMSWVRQ APGKGLEWVSAINSGGDNTYYADSVKGRFTISRDNSKNTL YLQMNSLRAEDTAVYYCARGPKLCFSSWGQGTLVTVSS |
| 64 | h_Nb1_2 | QVQLVESGGGLVQPGGSLRLSCAASGFTFSSYAMSWVRQ APGKGLEWVSDINSGGDNTDYADSVKGRFTISRDNAKNT LYLQMNSLRAEDTAVYYCARGPKLCFSSWGQGTQVTVSS |
| 65 | h_Nb1_3 | QVQLVESGGGLVQPGGSLRLSCAASGFTFSSYAMSWVRQ APGKGLEWVSDINSGGDNTDYADSVKGRFTISRDNSKNTL YLQMNSLRAEDTAVYYCARGPKLCFSSWGQGTQVTVSS |
| 66 | h_Nb1_4 | QVQLVESGGGLVQPGGSLRLSCAASGFTFSSYAMSWVRQ APGKGLEWVSDINSGGDNTDYADSVKGRFTISRDNAKNT LYLQMNSLKAEDTAVYYCARGPKLCFSSWGQGTQVTVSS |
| 67 | h_Nb1_5 | QVQLVESGGGLVQPGGSLRLSCAASGFTFSSYAMSWVRQ APGKGLEWVSDINSGGDNTDYADSVKGRFTISRDNSKNTL YLQMNSLKAEDTAVYYCARGPKLCFSSWGQGTQVTVSS |
| 68 | h_Nb2_1 | QVQLVESGGGLVQPGGSLRLSCAASGFTFGDRWMYWVR QAPGKGLEWVSAINPAGRSTYYADSVKGRFTISRDNSKNT LYLQMNSLRAEDTAVYYCTKDPGGYQWGQGTLVTVSS |
| 69 | h_Nb2_2 | QVQLLESGGGLVQPGGSLRLSCAASGFTFGDRWMYWLRQ APGKGLEWVSSINPAGRSTYYADSVKGRFTISRDNSKNTL YLQMNSLRAEDTAVYYCTKDPGGYQKGQGTQVTVSS |
| 70 | h_Nb2_3 | QVQLVESGGGLVQPGGSLRLSCAASGFTFGDRWMYWLR QAPGKGLEWVSSINPAGRSTYYADSVKGRFTISRDNAKNS LYLQMNSLRAEDTAVYYCTKDPGGYQKGQGTQVTVSS |
| 71 | h_Nb2_4 | QVQLLESGGGLVQPGGSLRLSCAASGFTFGDRWMYWVR QAPGKGLEWVSSINPAGRSTYYADSVKGRFTISRDNSKNT LYLQMNSLRAEDTAVYYCTKDPGGYQKGQGTQVTVSS |
| 72 | h_Nb2_5 | QVQLVESGGGLVQPGGSLRLSCAASGFTFGDRWMYWVR QAPGKGLEWVSSINPAGRSTYYADSVKGRFTISRDNAKNS LYLQMNSLRAEDTAVYYCTKDPGGYQKGQGTQVTVSS |
| 73 | h_Nb4_1 | QVQLVESGGGLVQPGGSLRLSCAASSRMFSGYTMGWVRQ APGKGLEWVSAIANGGFTYYADSVKGRFTISRDNSKNTLY LQMNSLRAEDTAVYYCNLVLVNGIPTRNWGQGTLVTVSS |
| 74 | h_Nb4_2 | QVQLLESGGGLVQPGGSLRLSCAASSRMFSGYTMGWYRQ APGKGLEWVAQIANGGFTDYIDSVKGRFTISRDNSKNTMY LQMNSLRAEDTAVYYCNLVLVNGIPTRNWGQGTQVTVSS |
| 75 | h_Nb5_1 | QVQLVESGGGLVQPGGSLRLSCAASGRDLMFYDVAWVR QAPGKGLEWVSAIRSSGTLTYYADSVKGRFTISRDNSKNT LYLQMNSLRAEDTAVYYCAARLQSATSHSDLREDEFNSW GQGTLVTVSS |
| 76 | h_Nb5_2 | QVQLQESGGGAVQSGESLRLSCAASGRDLMFYDVAWFRQ APGKEREGVSYIRSSGTLTKYADSVKGRFTISRDNDKNTV SLQMNSLNPEDTAKYYCAARLQSATSHSDLREDEFNSWG QGTQVTVSS |
| 77 | h_Nb5_3 | QVQLVESGGGLVQPGGSLRLSCAASGRDLMFYDVAWVR QAPGKGLEWVSYIRSSGTLTKYADSVKGRFTISRDNSKNT VYLQMNSLRAEDTAVYYCAARLQSATSHSDLREDEFNSW GQGTQVTVSS |
| 78 | h_Nb6_1 | QVQLVESGGGLVQPGGSLRLSCAASGFTFSSYAMSWVRQ APGKGLEWVSAIYRGGSDTYYADSVKGRFTISRDNSKNTL YLQMNSLRAEDTAVYYCIQGWGLGYANYDYWGQGTLVT VSS |
| 79 | h_Nb6_2 | QVQLLESGGGLVQPGGSLRLSCAASGFTFSSYAMSWARQ APGKGLEWVAGIYRGGSDTYYADSVKGRFTISRDNSKNTL YLQMNSLRAEDTAVYYCIQGWGLGYANYDYWGQGTQV TVSS |
| 80 | h_Nb9_1 | QVQLVESGGGLVQPGGSLRLSCAASGSIFGLGVKGWVRQ APGKGLEWVSAIISGGRIYYADSVKGRFTISRDNSKNTLYL QMNSLRAEDTAVYYCNAWGPGQRNYWGQGTLVTV |
| 81 | h_Nb9_2 | QVQLVESGGGLVQPGGSLRLSCASSGSIFGLGVKGWVRQ APGKGLEWVAQIISGGRISYADSVKGRFTISRDNAKNTVY LQMNSLRAEDTAVYYCNAWGPGQRNYWGRGTQVTVSS |
| 82 | h_Nb12_1 | QVQLVESGGGLVQPGGSLRLSCAASGRTFSSRAMGWVRQ APGKGLEWVSAISGSGRNTYYADSVKGRFTISRDNSKNTL YLQMNSLRAEDTAVYYCNAAGGGITIATMTQRTQYDYW GQGTLVTVSS |
| 83 | h_Nb12_2 | QVQLVESGGGLVQPGGSLRLSCAASGRTFSSRAMGWFRQ APGKELEFVAAISGSGRNTYYADSVKGRFTISRDNAKNSV YLQMNSLRAEDTAVYYCNAAGGGITIATMTQRTQYDYW GQGTQVTVSS |
| 84 | h_Nb13_1 | QVQLVESGGGLVQPGGSLRLSCAASGSIFSINAMGWVRQA PGKGLEWVSAITSGGSTYYADSVKGRFTISRDNSKNTLYL QMNSLRAEDTAVYYCNVERRRPPHYLSEYDFDWGQGTL VTVSS |
| 85 | h_Nb13_2 | EVQLVESGGGLVQPGGSLRLSCAASGSIFSINAMGWVRQA PGKGLEWVAAITSGGSTNYADSVKGRFTISRDNAKNTVYL QMNSLRAEDTAVYYCNVERRRPPHYLSEYDFDWGQGTQ VTVSS |
| 86 | h_Nb19_1 | QVQLVESGGGLVQPGGSLRLSCAASGRTFSGYAMSWVRQ APGKGLEWVSAISGSGYYADSVKGRFTISRDNSKNTLYLQ |
| | | MNSLRAEDTAVYYCAAAGEGITIATMTQRTQYDYWGQG TLVTVSS |
| 87 | h_Nb19_2 | QVQLVESGGGLVQPGGSLRLSCAASGRTFSGYAMAWFRQ APGKGREFVAAISGSGGRTYYADSRFTISRDNAKNTLYLQ MNSLRAEDTAVYYCAAAGEGITIATMTQRTQYDYWGQG TQVTVSS |
| 88 | h_Nb26_1 | QVQLVESGGGLVQPGGSLRLSCAASGSIFRINAMGWVRQ APGKGLEWVSAITSGGSTYYADSVKGRFTISRDNSKNTLY LQMNSLRAEDTAVYYCNVERRRPPHYYSDYDFDWGQGT LVTVSS |
| 89 | h_Nb26_2 | QVQLVQSGAEVKKPGASVKVSCKASGSIFRINAMGWYRQ APGQGLEWVAAITSGGSTNYADSVQGRFTITRDNSTSTVY LELRSLRSDDTAVYYCNVERRRPPHYYSDYDFDWGQGTQ VTVSS |
| 90 | h_Nb30_1 | QVQLVESGGGLVQPGGSLRLSCAASGFTFSNYYMSWVRQ APGKGLEWVSALNPSSSERYYADSVKGRFTISRDNSKNTL YLQMNSLRAEDTAVYYCARHLKYNWAPNYDYWGQGTL VTVSS |
| 91 | h_Nb30_2 | EVQLVESGGGLVQPGGSLRLSCAASGFTFSNYYMTWVRQ APGKGLEWVSFLNPSSSERFYADSVKGRFTISRDNAKNTL DLQMNSLRAEDTAVYYCARHLKYNWAPNYDYWGQGTQ VTVSS |

### Example 16: Optimization of CDR affinity maturation of nanobody

Full-length amino acid sequences (SEQ ID NO.21 and SEQ ID NO.25) of the human PD-L1 specifically bound nanobodies according to the above embodiments were selected, and the antibody CDR region and the FR region thereof were annotated by the CCG method; by means of antibody homologous modeling, a suitable FR region and CDR region template were selected, and the optimal three-dimensional protein structure of the nanobodywas constructed and selected; a human PD-L1 protein crystal structure was obtained from a PDB protein database, and a preferred PD-L1 nanobody-PD-L1 protein complex structure candidate library was obtained by means of protein complex homologous modeling and a protein-protein molecule docking method; an optimal docking conformation according to the PD-1/PD-L1 *in vitro* competition binding feature of the preferred antibody was determined, preferably a suitable docking angle; interaction key sites for the selected nanobody-PD-L1 protein complex structure was analyzedwith paying attention to Van der Waals force, hydrogen bonds, ion bonds, hydrophobic effects and the like; and the action site pair with a lower affinity energy value between the nanobody and the active site on the PD-L1 as an affinity mature point mutation object was focused on; a nanobody-PD-L1 interaction residue pair was selected to meet the above conditions, point mutation on the corresponding amino acid site of the nanobody was performed, the amino acid sites into other 19 natural amino acids were respectively tested and mutated, the change of the interaction force between the mutation rear site and the corresponding site on the PD-L1 were analyzed, and a mutation mode enabling the affinity to be remarkably improved was seleced; point mutation screening on sites having weak interaction with the PD-L1 protein on the CDR region of the nanobody was performed, and an excellent mutation combination was selected; obtaining sequences produced by computer-level screening of candidate affinity maturation solution through gene synthesis, cloning, transfection and protein expression purification, and comparing the affinity level of the candidate antibody and the original antibody (SEQ ID NO.21 and SEQ ID NO.25) with PD-L1 and blocking PD-1/PD-L1 activity by means of an ELISA method; and finally determining a CDR region affinity maturation scheme of the preferred antibody, wherein the sequences of the antibody after affinity maturation are SEQ ID NO.92 - SEQ ID NO.117. Wherein, the original antibody corresponding to the sequence shown in SEQ ID NO.92 -106 is h_ Nb1 VHH (SEQ ID NO.21); and the original antibody corresponding to the sequence shown in SEQ ID NO.107 -117 is h_ Nb2 VHH (SEQ ID NO.25).

**Table 6: VHH sequence after affinity maturation of anti-human PD-L1 antibody**

| SEQ ID NO. | Name | Sequence | Increased relative affinity |
|---|---|---|---|
| 92 | h_Nb1_ A61N | DVQLQESGGGLVQPGGSLRLSCAASGFTFSSYAMS WVRQAPGKGLEWVSDINSGGDNTDYNDSVKGRF TISRDNAKNTLYLQMNSLKPEDTAVYYCARGPKL CFSSWGQGTQVTVSS | 5.1327 |
| 93 | h_Nb1_ A61Q | DVQLQESGGGLVQPGGSLRLSCAASGFTFSSYAMS WVRQAPGKGLEWVSDINSGGDNTDYQDSVKGRF TISRDNAKNTLYLQMNSLKPEDTAVYYCARGPKL CFSSWGQGTQVTVSS | 4.6638 |
| 94 | h_Nb1_ A61K | DVQLQESGGGLVQPGGSLRLSCAASGFTFSSYAMS WVRQAPGKGLEWVSDINSGGDNTDYKDSVKGRF TISRDNAKNTLYLQMNSLKPEDTAVYYCARGPKL CFSSWGQGTQVTVSS | 4.1233 |
| 95 | h_Nb1_ | DVQLQESGGGLVQPGGSLRLSCAASGFTFSSYAMS | 4.2832 |
| | D59R | WVRQAPGKGLEWVSDINSGGDNTRYADSVKGRF TISRDNAKNTLYLQMNSLKPEDTAVYYCARGPKL CFSSWGQGTQVTVSS | |
| 96 | h_Nb1_ D59M | DVQLQESGGGLVQPGGSLRLSCAASGFTFSSYAMS WVRQAPGKGLEWVSDINSGGDNTMYADSVKGRF TISRDNAKNTLYLQMNSLKPEDTAVYYCARGPKL CFSSWGQGTQVTVSS | 4.9025 |
| 97 | h_Nb1_ D59F | DVQLQESGGGLVQPGGSLRLSCAASGFTFSSYAMS WVRQAPGKGLEWVSDINSGGDNTFYADSVKGRFT ISRDNAKNTLYLQMNSLKPEDTAVYYCARGPKLC FSSWGQGTQVTVSS | 5.1213 |
| 98 | h_Nb1_ D62W | DVQLQESGGGLVQPGGSLRLSCAASGFTFSSYAMS WVRQAPGKGLEWVSDINSGGDNTDYAWSVKGRF TISRDNAKNTLYLQMNSLKPEDTAVYYCARGPKL CFSSWGQGTQVTVSS | 11.6979 |
| 99 | h_Nb1_ D62Y | DVQLQESGGGLVQPGGSLRLSCAASGFTFSSYAMS WVRQAPGKGLEWVSDINSGGDNTDYAYSVKGRF TISRDNAKNTLYLQMNSLKPEDTAVYYCARGPKL CFSSWGQGTQVTVSS | 5.7977 |
| 100 | h_Nb1_ C103K | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARGPKL KFSSWGQGTQVTVSS | 3.3129 |
| 101 | h_Nb1_ C103M | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARGPKL MFSSWGQGTQVTVSS | 3.3768 |
| 102 | h_Nb1_ C103W | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARGPKL WFSSWGQGTQVTVSS | 3.8328 |
| 103 | h_Nb1_ G99R | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARRPKL CFSSWGQGTQVTVSS | 12.1151 |
| 104 | h_Nb1_G99Q | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARQPKL CFSSWGQGTQVTVSS | 6.1589 |
| 105 | h_Nb1_ G99W | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARWPKL CFSSWGQGTQVTVSS | 6.1572 |
| 106 | h_Nb1_ G99Y | DVQLQESGGGLVQPGGSLRLSCAASGFTFSSYAMS WVRQAPGKGLEWVSDINSGGDNTDYADSVKGRF TISRDNAKNTLYLQMNSLKPEDTAVYYCARYPKL CFSSWGQGTQVTVSS | 7.0186 |
| 107 | h_Nb2_ G30R | DVQLQESGGGLVQPGGSLRLSCAASGFTFRDRWM YWLRQAPGKGLEWVSSINPAGRSTYYADSVKGRF TISRDNAKNTLYLQMNSLKSEDTAVYYCTKDPGG YQKGQGTQVTVSS | 6.2276 |
| 108 | h_Nb2_ G30F | DVQLQESGGGLVQPGGSLRLSCAASGFTFFDRWM YWLRQAPGKGLEWVSSINPAGRSTYYADSVKGRF TISRDNAKNTLYLQMNSLKSEDTAVYYCTKDPGG YQKGQGTQVTVSS | 5.1098 |
| 109 | h_Nb2_ G30W | DVQLQESGGGLVQPGGSLRLSCAASGFTFWDRW MYWLRQAPGKGLEWVSSINPAGRSTYYADSVKG RFTISRDNAKNTLYLQMNSLKSEDTAVYYCTKDP GGYQKGQGTQVTVSS | 10.0189 |
| 110 | h_Nb2_ G101R | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDPRG YQKGQGTQVTVSS | 6.4982 |
| 111 | h_Nb2_ G101W | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDPWG YQKGQGTQVTVSS | 5.9213 |
| 112 | h_Nb2_ G101Y | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDPGY YQKGQGTQVTVSS | 8.4837 |
| 113 | h_Nb2_ F29R | DVQLQESGGGLVQPGGSLRLSCAASGFTRGDRWM YWLRQAPGKGLEWVSSINPAGRSTYYADSVKGRF TISRDNAKNTLYLQMNSLKSEDTAVYYCTKDPGG YQKGQGTQVTVSS | 1.4558 |
| 114 | h_Nb2_ F29W | DVQLQESGGGLVQPGGSLRLSCAASGFTWGDRW MYWLRQAPGKGLEWVSSINPAGRSTYYADSVKG RFTISRDNAKNTLYLQMNSLKSEDTAVYYCTKDP GGYQKGQGTQVTVSS | 1.4356 |
| 115 | h_Nb2_ P100R | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDRGG YQKGQGTQVTVSS | 7.2081 |
| 116 | h_Nb2_ P100Q | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDQGG YQKGQGTQVTVSS | 3.4102 |
| 117 | h_Nb2_ P100E | DVQLQESGGGLVQPGGSLRLSCAASGFTFGDRWM YWLRQAPGKGLEWVSSINPAGRSTYYADSVKGRF TISRDNAKNTLYLQMNSLKSEDTAVYYCTKDEGG YQKGQGTQVTVSS | 3.9779 |

### Discussion

Existing PD-L1 antibodies have good targeting and inhibitory effects on high immunogenicity " hot " tumors and tumors with high PD-L1 expression level, and have poor inhibition effects for tumors with low immunogenicity and low expression of PD-L1. Therefore, the combined treatment scheme can be based on improving the immunogenicity of the tumor and the expression level of PD-L1, so as to improve the treatment effect and response rate.

A scientific, reasonable, safe and effective combined treatment scheme can also guide the design of novel drug molecules. On the basis of combined treatment of PD-L1 nanobodies and TLR7 agonists, tumor growth can be significantly inhibited. PD-L1 and TLR7 double-targeting nanobody coupling drugs are developed, and its efficacy is superior to that of combined treatment. It can coordinate the innate immune and adaptive immune responses, target and reshape the tumor immune microenvironment, and improve the expression level of PD-L1 in tumor tissues, so that extremely significant anti-tumor activity and response rate are also displayed in "cold" tumors and tumor models with low expression of PD-L1, showing its clinical application value.

The present invention provides a PD-L1 and TLR7 double-targeting nanobody coupling drug for the first time, which can subsequently develop tumor immunotherapy drugs, and is particularly suitable for tumors with low immunogenicity and/or low expression of PD-L1.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. An antibody-drug conjugate or a pharmaceutically acceptable salt thereof, wherein the structure of the antibody-drug conjugate is as shown in formula I:
Ab-(J-U)n (I)
wherein,
Ab is a PD-L1 antibody;
U is each independently a TLR agonist;
J is a chemical bond or linker;
n is 0 or a positive integer;
"-"is a chemical bond or linker or connector.

2. The antibody-drug conjugate or a pharmaceutically acceptable salt thereof according to claim 1, wherein the PD-L1 antibody is a PD-L1 nanobody or a derivative antibody thereof, wherein the PD-L1 nanobody specifically binds to PD-L1, and the complementarity determining region CDR of the VHH chain in the nanobody is selected from the following group:
(1) CDR1 shown in SEQ ID NO: 2, CDR2 shown in SEQ ID NO: 3, CDR3 shown in SEQ ID NO: 4;
(2) CDR1 shown in SEQ ID NO:6, CDR2 shown in SEQ ID NO:7, CDR3 shown in SEQ ID NO:8;
(3) CDR1 shown in SEQ ID NO:10, CDR2 shown in SEQ ID NO:11, CDR3 shown in SEQ ID NO:12;
(4) CDR1 shown in SEQ ID NO:14, CDR2 shown in SEQ ID NO:15, CDR3 shown in SEQ ID NO:16;
(5) CDR1 shown in SEQ ID NO:18, CDR2 shown in SEQ ID NO:19, CDR3 shown in SEQ ID NO:20;
(6) CDR1 shown in SEQ ID NO:22, CDR2 shown in SEQ ID NO:23, CDR3 shown in SEQ ID NO:24;
(7) CDR1 shown in SEQ ID NO:26, CDR2 shown in SEQ ID NO:27, CDR3 shown in SEQ ID NO:28;
(8) CDR1 shown in SEQ ID NO:30, CDR2 shown in SEQ ID NO:31, CDR3 shown in SEQ ID NO:32;
(9) CDR1 shown in SEQ ID NO:34, CDR2 shown in SEQ ID NO:35, CDR3 shown in SEQ ID NO:36;
(10) CDR1 shown in SEQ ID NO:22, CDR2 shown in SEQ ID NO:38, CDR3 shown in SEQ ID NO:39;
(11) CDR1 shown in SEQ ID NO:41, CDR2 shown in SEQ ID NO:42, CDR3 shown in SEQ ID NO:43;
(12) CDR1 shown in SEQ ID NO:45, CDR2 shown in SEQ ID NO:46, CDR3 shown in SEQ ID NO:47;
(13) CDR1 shown in SEQ ID NO:49, CDR2 shown in SEQ ID NO:50, CDR3 shown in SEQ ID NO:51;
(14) CDR1 shown in SEQ ID NO:53, CDR2 shown in SEQ ID NO:54, CDR3 shown in SEQ ID NO:55;
(15) CDR1 shown in SEQ ID NO:57, CDR2 shown in SEQ ID NO:50, CDR3 shown in SEQ ID NO:58; and
(16) CDR1 shown in SEQ ID NO:60, CDR2 shown in SEQ ID NO:61, CDR3 shown in SEQ ID NO:62.

3. An antibody-drug conjugate or a pharmaceutically acceptable salt thereof according to claim 1, wherein the TLR agonist is a TLR7 agonist.

4. An antibody-drug conjugate or a pharmaceutically acceptable salt thereof according to claim 3, wherein the TLR7 agonist comprises: SZU-101:

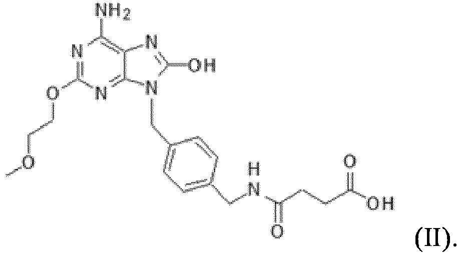

5. An antibody-drug conjugate or a pharmaceutically acceptable salt thereof according to claim 1, wherein the antibody-drug conjugate or a pharmaceutically acceptable salt thereof are used for preaparing a composition or formulation, which is used for:
(a) promoting maturation of dendritic cells;
(b) increasing the function of tumor infiltrating cytotoxic cells (CD8 + T cells and NK cells);
(c) promoting the expression of granzyme B and IFN-γ in tumor infiltrating cytotoxic cell;
(d) promoting re-polarization of tumor-associated macrophages;
(e) reducing the infiltration of TGF-β + macrophages;
(f) promoting the infiltration of IFN-γ + CD4 + T cells;
(g) promoting the expression of PD-L1 by macrophages in tumors;
(h) targeting and remodeling a tumor immune microenvironment;
(i) increase the PD-L1 level of tumor cells; and/or
(j) treating the tumor with moderate-expression or low expression of PD-L1.

6. A pharmaceutical composition, wherein the pharmaceutical composition comprises:
(a) an antibody-drug conjugate or a pharmaceutically acceptable salt thereof according to claim 1;
(b) a pharmaceutically acceptable carrier.

7. A pharmaceutical composition according to claim 6, wherein the pharmaceutical composition is used to treat tumors with low PD-L1 expression.

8. A method for preventing or treating tumors, by administrating the nanobody conjugate drug according to claim 1 to a subject in need thereof.

9. A PD-L1 nanoboy, which can specifically bind to PD-L1, and the complementary determination region CDR of the VHH chain in the nanobody is selected one or more from the group consisting of:
(1) CDR1 shown in SEQ ID NO: 2, CDR2 shown in SEQ ID NO: 3, CDR3 shown in SEQ ID NO: 4;
(2) CDR1 shown in SEQ ID NO:6, CDR2 shown in SEQ ID NO:7, CDR3 shown in SEQ ID NO:8;
(3) CDR1 shown in SEQ ID NO:10, CDR2 shown in SEQ ID NO:11, CDR3 shown in SEQ ID NO:12;
(4) CDR1 shown in SEQ ID NO:14, CDR2 shown in SEQ ID NO:15, CDR3 shown in SEQ ID NO:16;
(5) CDR1 shown in SEQ ID NO:18, CDR2 shown in SEQ ID NO:19, CDR3 shown in SEQ ID NO:20;
(6) CDR1 shown in SEQ ID NO:22, CDR2 shown in SEQ ID NO:23, CDR3 shown in SEQ ID NO:24;
(7) CDR1 shown in SEQ ID NO:26, CDR2 shown in SEQ ID NO:27, CDR3 shown in SEQ ID NO:28;
(8) CDR1 shown in SEQ ID NO:30, CDR2 shown in SEQ ID NO:31, CDR3 shown in SEQ ID NO:32;
(9) CDR1 shown in SEQ ID NO:34, CDR2 shown in SEQ ID NO:35, CDR3 shown in SEQ ID NO:36;
(10) CDR1 shown in SEQ ID NO:22, CDR2 shown in SEQ ID NO:38, CDR3 shown in SEQ ID NO:39;
(11) CDR1 shown in SEQ ID NO:41, CDR2 shown in SEQ ID NO:42, CDR3 shown in SEQ ID NO:43;
(12) CDR1 shown in SEQ ID NO:45, CDR2 shown in SEQ ID NO:46, CDR3 shown in SEQ ID NO:47;
(13) CDR1 shown in SEQ ID NO:49, CDR2 shown in SEQ ID NO:50, CDR3 shown in SEQ ID NO:51;
(14) CDR1 shown in SEQ ID NO:53, CDR2 shown in SEQ ID NO:54, CDR3 shown in SEQ ID NO:55;
(15) CDR1 shown in SEQ ID NO:57, CDR2 shown in SEQ ID NO:50, CDR3 shown in SEQ ID NO:58; and
(16) CDR1 shown in SEQ ID NO:60, CDR2 shown in SEQ ID NO:61, CDR3 shown in SEQ ID NO:62.

10. A medicine box, wherein the medicine box comprises:
(1) a first container, and a PD-L1 nanobody according to claim 9 located in the first container, and a pharmaceutically acceptable carrier;
(2) a second container, and a TLR7 agonist located in the second container, and a pharmaceutically acceptable carrier;
and (3) optional operation instruction.

11. An immunoconjugate containing:
(a) the PD-L1 nanobody according to claim 9; and
(b) other coupling parts.

12. A fusion protein, wherein the fusion protein comprises:
(a) the PD-L1 nanobody according to claim 9; and
(b) optional peptide molecules and protein fragments with therapeutic properties.

13. A multispecific antibody, wherein the multispecific antibody comprises:
(a) the PD-L1 nanobody according to claim 9; and
(b) optional antibody molecule targeting the second antigen.

14. A method for preparing the antibody-drug conjugate according to claim 1, which comprises the steps:
configuring a reaction system, the reaction system comprising an antibody and a free drug molecule, and then performing a coupling reaction to obtain the antibody-drug conjugate, wherein the drug molecule comprises a TLR agonist and a linker.
